# EUROPEAN PATENT SPECIFICATION

(11) **EP 0 548 095 B1**
(45) Date of publication and mention of the grant of the patent: **26.11.1997**
(21) Application number: 91914597.9
(22) Date of filing: 02.08.1991
(51) Int. Cl.: C12N 15/53, A61K 38/44, C12N 9/02, C12N 15/71

(54) **PHARMACEUTICAL COMPOSITIONS CONTAINING SUPEROXIDE DISMUTASE FROM BACILLUS STEAROTHERMOPHILUS AND BACILLUS CALDOTENAX**
PHARMAZEUTISCHE ZUSAMMENSETZUNGEN DIE SUPEROXIDDISMUTASE AUS BACILLUS STEAROTHERMOPHILUS UND BACILLUS CALDOTENAX ENTHALTEN
COMPOSITIONS PHARMACEUTIQUES CONTENANT UNE DISMUTASE DE SUROXYDE DE BACILLUS STEAROTHERMOPHILUS ET BACILLUS CALDOTENAX

(30) Priority: 03.08.1990 GB 9017037
(43) Date of publication of application: 30.06.1993
(73) Proprietor: Microbiological Research Authority, Salisbury, Wiltshire SP4 0JG (GB)
(72) Inventor: ATKINSON, Anthony, Porton Down, Salisbury, Wilts SP4 0JG (GB); BOWN, Kevin John, Porton Down, Salisbury, Wilts SP4 0JG (GB); BREHM, John Karl, Porton Down, Salisbury, Wilts SP4 0JG (GB); CHAMBERS, Stephen Phillip, Porton Down, Salisbury, Wilts SP4 0JG (GB); MINTON, Nigel Peter, Porton Down, Salisbury, Wilts SP4 0JG (GB)
(74) Representative: Ritter, Stephen David
(86) International application number: GB9101325
(87) International publication number: WO9202625

(56) References cited:
- EP-A- 172 577
- EP-A- 289 667
- EP-A- 314 274
- US-A- 4 820 642
- WPIL, Derwent Abstracts, 1988, Derwent Publications Ltd., London (GB); AN 88- 333484
- BIOCHEMISTRY, vol. 19, no. 13, Easton, PA (US); C.J. BROCK et al., pp. 2873- 2882
- JOURNAL OF BACTERIOLOGY, vol. 172, no. 3, March 1990; C. BOWLER et al., pp. 1539-1546
- JOURNAL OF GEN. APPL. MICROBIOLOGY, vol. 22, 1976, Tokyo (JP); A.A. YOUSTEN et al., pp. 161-164
- AGRICULTURAL & BIOLOGICAL CHEMISTRY, vol. 47, no. 12, December 1983, Tokyo (JP); K. TSUKUDA et al, pp. 2865-2870

## Description

This invention relates to a process for producing enzymes having superoxide dismutase activity, novel superoxide dismutase enzymes and novel pharmaceutical compositions comprising enzymes having superoxide dismutase activity.

One consequence of oxidative metabolism is the generation of superoxide radicals (O₂⁻), which mediate extensive damage to the cellular components of living organisms. The molecular dismutation of O₂⁻ to hydrogen peroxide (H₂O₂) and oxygen (O₂) is catalysed by a ubiquitous class of metalloenzymes termed superoxide dismutases (SODs). Other abbreviations appear in the appendix preceding the figure legends. The prevalence of SODs in all living organisms which tolerate exposure to molecular O₂⁻ has led to the compelling suggestion that these enzymes form the first line of the cell's defence against oxygen damage (Fridovich, 1975).

On the basis of their metal ion content, three classes of SOD are recognised: Cu/Zn-, Fe-, and Mn-containing enzymes. While all three forms catalyse the same reaction, the Fe-containing SODs (FeSOD) are largely confined to prokaryotes and the Cu/Zn enzymes (Cu/ZnSOD) predominantly to eukaryotes. Mn-containing SODs (MnSOD) are universally present. In eukaryotes MnSODs are localised to the mitochondria, while the Cu/ZnSODs reside in the cytosol (Geller and Winge, 1984). Escherichia coli contains three isoenzymatic forms of SOD: a MnSOD (sodA), a FeSOD (sodB) and a hybrid enzyme containing both manganese and iron (Hassan and Fridovich, 1977).

SODs from various sources are currently of great interest as potential therapeutic treatments for oxidative damage. Their use in a clinical setting for the treatment of a wide variety of disorders has been proposed (see Beck et al., 1988).

These include: (i) prevention of oncogenesis, tumour promotion and invasiveness, and UV-induced damage; (ii) protection of cardiac tissue against post-ischemia reperfusion damage; (iii) as an antiinflamatory agent; (iv) to reduce the cytotoxic and cardiotoxic effects of anticancer drugs, and; (v) to improve the longevity of living cells. Indeed, currently bovine Cu/ZnSOD is being utilised for the treatment of inflamed tendons in horses and for treating osteoarthritis in man (Puhl et al., 1984).

SODs currently proposed for therapy suffer the severe disadvantage of being highly immunogenic and consequently, as a result of the antibody response produced on administration, have proved to be of low clinical utility. Further available SODs, particularly those from mammalian sources, are difficult to obtain in large amounts in view of their low concentration in mammalian cells and the tedious isolation procedures required to produce them in satisfactory levels of purity.

Thus for example EP-A-O 172 577 (Takeda) describes the pharmaceutical use of the MnSOD of Serratia marcescens as an antiinflammatory agent, but the only dosage forms described are enteric capsules and tablets. Similarly JP-A-63245671 (Shobo K.K. and Unichika K.K.) suggests the pharmaceutical use of a modified MnSOD of Bacillus stearothermophilus in the cosmetic and pharmaceutical fields. The unmodified enzyme is stated to be unsuitable for use due to its antigenicity and the specification prescribes a modification using a polyalkylene glycol.

GB-A-2183658 describes the expression of human MnSOD in E.coli and proposes various pharmacological uses for the resulting product. Also the pharmaceutical use of the SOD of Streptococcus lactis is described in EP-A-O 289 667.

Available SOD enzymes suffer disadvantages limiting their clinical utility, including a relatively short half-life in solution, loss of activity at pHs below pH7 and high antigenicity. Hitherto it has consequently not been possible to produce effective pharmaceutical compositions for countering the adverse effects associated with the presence in tissues of superoxide radicals.

It has now been surprisingly found that the MnSOD enzymes of *B.stearothermophilus* (BS) and *B.caldotenax* (BC) when in native, i.e. chemically unmodified form, have a significantly lower antigenicity than those derived from eukaryotic cells and can be used with greater therapeutic effect.

Contrary to the teaching of JP-A-63245671 the BS and BC MnSOD enzymes used for producing pharmaceutical compositions according to the invention have been found to be essentially non-antigenic in native form. The suggestion in JP-A-63245671 that BS MnSOD is highly antigenic may be a result of the highly impure nature of the enzyme used in the described procedures. According to our findings, both the BS and BC enzyme, either when subjected to purification procedures so as to remove pyrogenic impurities associated with cell components of the BS and BC organisms, or if produced by recombinant techniques which necessarily avoid the presence of such impurities, are essentially non-antigenic (to the limits of available immunoassay techniques).

Thus according to one aspect of the invention, there are provided pharmaceutical compositions for use in the prophylaxis and/or treatment of pathological conditions resulting from the presence of superoxide radicals, comprising an MnSOD enzyme and a pharmaceutically acceptable excipient, characterised in that the MnSOD enzyme is (i) is in native form, (ii) catalyses the dismutation of superoxide radicals, and (iii) has (a) the amino acid sequence of *Bacillus Stearothermophilus* (BS) or *Bacillus Caldotenax* (BC) MnSOD or (b) a variant sequence that differs form the sequences in (a) by from 1 to 20 amino acid insertions, deletions and/or substitutions.

Preferably the MnSOD enzyme in native form is essentially unmodified, chemically, compared to the MnSOD enzyme present in a culture of a MnSOD-producing BC or BS microorganism.

The culture medium is desirably supplemented with manganese. In order to achieve high levels of expression of MnSOD >^{.}0.01mm manganese is generally required. Thus to obtain levels of expression of around 10% MnSOD (expressed as a percentage of total soluble protein), 0.01mM of manganese salt should be included.

It is preferred that pharmaceutical compositions according to the invention are substantially free of pyrogens consisting of macromolecular substances native to *B stearothermophilus* or *B caldotenax.* This may be achieved for example by producing the MnSOD enzyme by culturing a transformed microorganism being of a species other than *B stearothermophilus* or *B caldotenax.*

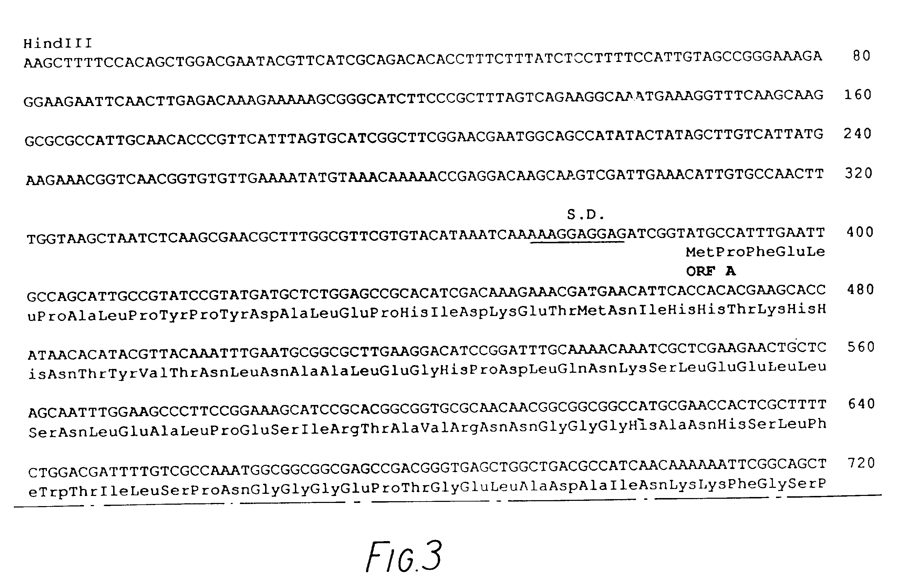

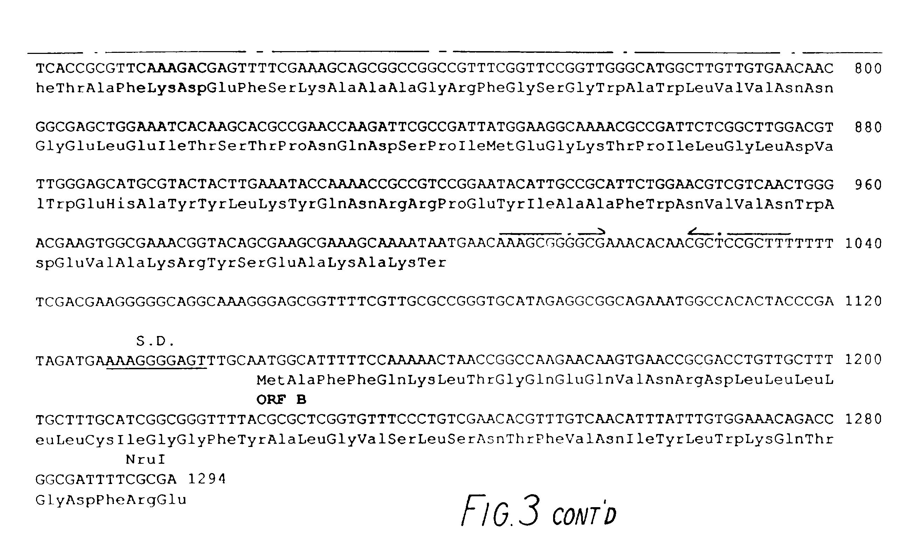

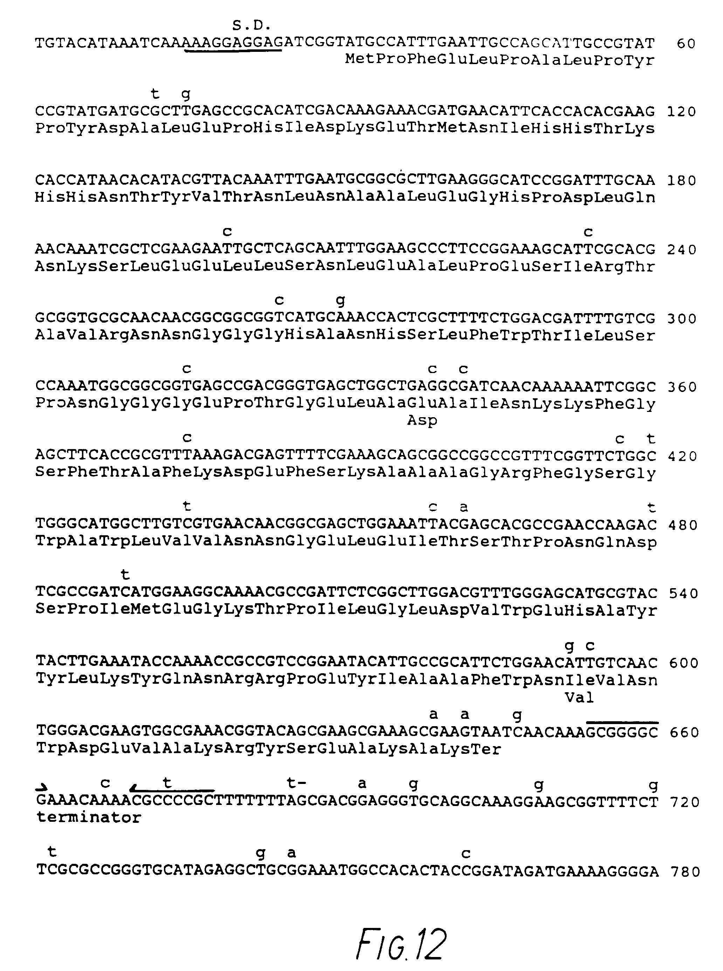

The MnSOD enzymes preferably have an amino acid sequence selected from
(i) the amino acid sequence depicted in Figure 12 for BC MnSOD,
(ii) the amino acid sequence depicted in Figure 3 for BS MnSOD, and
(iii) amino acid sequences which differ from the sequences (i) and (ii) by from 1 to 10 amino acid insertions, deletions and/or substitutions.

Most preferably amino acid sequences (iii) differ from the sequences (i) and (ii) by from 1 to 5, e.g. 1, 2 or 3 amino acid insertions, deletions and/or substitutions.

Pharmaceutical compositions according to the invention may be prepared in the form (a) of an injectable solution, or (b) a solution suitable for perfusing tissues during surgical or transplantation procedures. Such compositions typically contain from 0.001 to 1.0, preferably from 0.01 to 1.0 mg/l of said MnSOD enzyme, preferably from 0.01 to 1.0 mg/l of said MnSOD enzyme, most preferably from 0.05 to 0.5 mg/l of said MnSOD enzyme.

According to a further aspect of the invention there is provided a process for producing a pharmaceutical composition which comprises the steps of (a) culturing an MnSOD enzyme-producing microorganism so as to produce an MnSOD enzyme-containing culture, (b) isolating MnSOD enzyme from the culture, (c) purifying the isolated MnSOD enzyme so as to produce purified enzyme which is essentially unmodified, chemically, compared to the MnSOD enzyme present in the MnSOD-containing culture produced in step (a), and (d) mixing the chemically unmodified MnSOD enzyme with a pharmaceutically acceptable excipient, characterised in that said MnSOD enzyme (i) is in native form, (ii) catalyses the dismutation of superoxide radicals, and (iii) has (a) the amino acid sequence of *Bacillus Stearothermophilus* (BS) or *Bacillus Caldotenax* (BC) MnSOD or (b) a variant sequence that differs form the sequences in (a) by from 1 to 20 amino acid insertions, deletions and/or substitutions.

As indicated, it is preferred that the transformed microorganism referred to in step (a) is a transformed microorganism of a species other than *B stearothermophilus* or *B caldotenax.*

Further in accordance with the invention there is provided the use of an MnSOD enzyme according to the invention in the manufacture of a pharmaceutical composition for the prophylaxis and/or treatment of pathological conditions resulting From the presence of superoxide radicals.

BS and BC MnSOD enzymes have been found to be particularly useful in the manufacture of infusing solutions for organs undergoing surgery or transplantation.

Thus a more specific use in accordance with the invention comprises using of an MnSOD enzyme in the manufacture of an infusing solution for organs undergoing surgery or transplantation, especially such organs which are isolated from normal blood supply, the MnSOD enzyme being in native form and having substantially the amino acid sequence of BS or BC MnSOD.

The BC and BS MnSOD enzymes used in accordance with the invention have properties which provide distinct advantages compared to previously used SODs. Particularly
(a) Both BS and BC MnSODs have a half life in solution:
   of >2hrs at 60°C.
   of >30mins at 65°C
   at least 10 mins at 70°C
   at least 2 mins at 75°C
   at pH7.5 and an MnSOD protein concentration of 0.5mg/ml
(b) Both BC and BS MnSOD retain at least 10% of their full catalytic activity at pHs below 7 and above pH6.
(c) The antigenicity of both enzymes is so low as to be impossible to quantify. Thus whereas enzymes such as S.lactis and S.marcescens SODs have antigenicities which can be determined by assessing antibody titres in rabbits, normal protocols fail to elicit any antibody response for native BC or BS MnSODs either when produced by recombinant procedures or extracted from BC or BS, followed by purification to remove pyrogens.
(d) Both BC and BS MnSOD have a half life in sterile solution at pH7.5 and a protein concentration of 0.5mg/ml of >1 year at 4°C and >3 months at ambient (15-20°C). In 50% glycerol at -20°C (both enzymes still being in liquid state under these conditions) their half lives are in excess of 5 years.

In addition to the specific pharmacological uses described herein, the BS and BC MnSOD enzymes of the invention may be used industrially as follows:
(i) The generation of hydrogen peroxide in diagnostic assays. Many enzymes and reagents are available for estimating/monitoring hydrogen peroxide.
(ii) Removal of superoxide in industrial systems. Many superoxide scavengers are used in industry including perfumes, anaerobic processing etc.
(iii) Removal of superoxide (a taste destroyer and spoiler) in foods etc.

The MnSOD enzyme of Bacillus caldotenax has never been isolated or described hitherto and is a novel substance forming a further aspect of the present invention.

Thus the invention further provides an MnSOD enzyme being in substantially pure form and having essentially the amino acid sequence of *B caldotenax*, said amino acid sequence being selected from
(i) the amino acid sequence depicted in Figure 12,
(ii) amino acid sequences which differ from the sequence (i) by from 1 to 30 amino acid insertions, deletions and/or substitutions, with the proviso that said amino acid sequences (ii) have Glu in the location marked 103 and/or have Ile in the location marked 188.

Sequences (ii) may for example differ from the sequence (i) by from 1 to 20, preferably from 1 to 10 amino acid insertions, deletions and/or substitutions.

To date the structural genes encoding various SODs have been cloned from a number of different eukaryotic and prokaryotic sources, including the Cu/ZnSODs of man (Sherman et al., 1983), Saccharomyces cerevisiae (Bermingham et al., 1988), and Drosophila (Seto et al., 1989), the human MnSOD (McCord et al., 1977), the FeSOD of Anacystis nidulans (Laudenbach et al., 1989), and the MnSOD (Touati et al., 1983) and FeSOD (Sakamoto and Touati, 1984) of E.coli. The cloning of the MnSOD of BS and its expression in yeast has also been described (Bowler et al., 1990).

The present application further describes the cloning of the MnSOD of the Gram-positive thermophile *Bacillus caldotenax,* the determination of the entire nucleotide sequences of the MnSOD enzymes of both *Bacillus stearothermophilus* and *Bacillus caldotenax* and the over-expression of both enzymes in E.coli.

Thus the invention further provides a recombinant DNA molecule comprising a DNA sequence coding for a BC MnSOD enzyme as defined above.

Such recombinant DNA molecules may be selected from (a) the DNA coding sequence depicted in Figure 12 and (b) DNA sequences which are degenerate according to the genetic code to said sequence.

As indicated, we have now developed a procedure for overexpressing the MnSOD of BC and BS in E.coli which forms a further aspect of the present invention.

Thus according to a further aspect of the invention there is provided a process for producing an MnSOD enzyme which comprises culturing a transformed strain of E.coli containing a recombinant plasmid comprising at least one structural gene coding for an MnSOD enzyme operatively linked to a promoter, characterised in that said promoter is the native *trp* promoter of *E.coli*

Examples of specific promoter sequences are as follows:
(i) a promoter that has a base sequence comprising the sequence TTGACA at the 5' end and the sequence TTAACT at the 3' end.
(ii) a promoter that has a base sequence comprising the sequence TCAATT at the 5' end and the sequence ACAGTT at the 3' end.
(iii) a promoter that has the following intervening sequence located between said 3' and 5' sequences of (i) or (ii): ATTAATCATCGAACTAG
(iv) a promoter that has the following base sequence:

TTGACAATTAATCATCGAACTAGTTAACT

The sequence TTGACARTTAATCATCGAACTAGTTAACT may be preceded by the sequence GCTTACTCCCCATCCCCCCAGTGAATTCCCCTG and followed by the sequence AGTACGCAGCTTGGC.

The following protocol was adopted for the molecular cloning of the B. stearothermophilus sod gene.

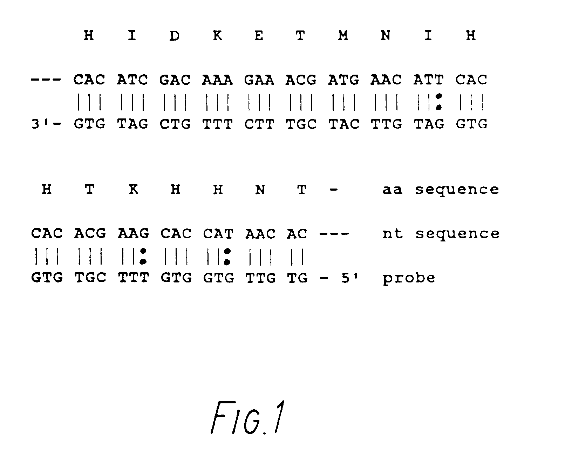

The first step in the cloning of the encoding gene was to design an oligonucleotide which demonstrated sufficient homology to the structural gene to allow its detection by DNA/DNA hybridisation experiments. Analysis of the amino acid sequence indicated that amino acids 17 through 34 represented a peptide exhibiting minimal translational degeneracy. Accordingly, a 50 mer antisense oligonucleotide was synthesised (Fig. 1) in which nucleotide bases used in positions of codon degeneracy corresponded to those most frequently used in B. stearothermophilus genes. To test that the synthesised oligonucleotide hybridised to a specific sequence in the B.stearothermophilus genome, Southern blot experiments were undertaken. The oligonucleotide was radiolabelled and used in DNA/DNA hybridisation reactions against B. stearothermophilus NCA1503 genomic DNA cleaved with various restriction enzymes.

Under the conditions employed (see the Examples below) the probe was shown to hybridise strongly to the following discrete restriction fragments; a 2.45 kb BclI, 5.1 kb ClaI, 6.8 kb EcoRI, 3.4 kb HindIII, 20 kb PstI, 3.2 kb SalI, 3.5 kb SstI and a 17 kb XhoI fragment.

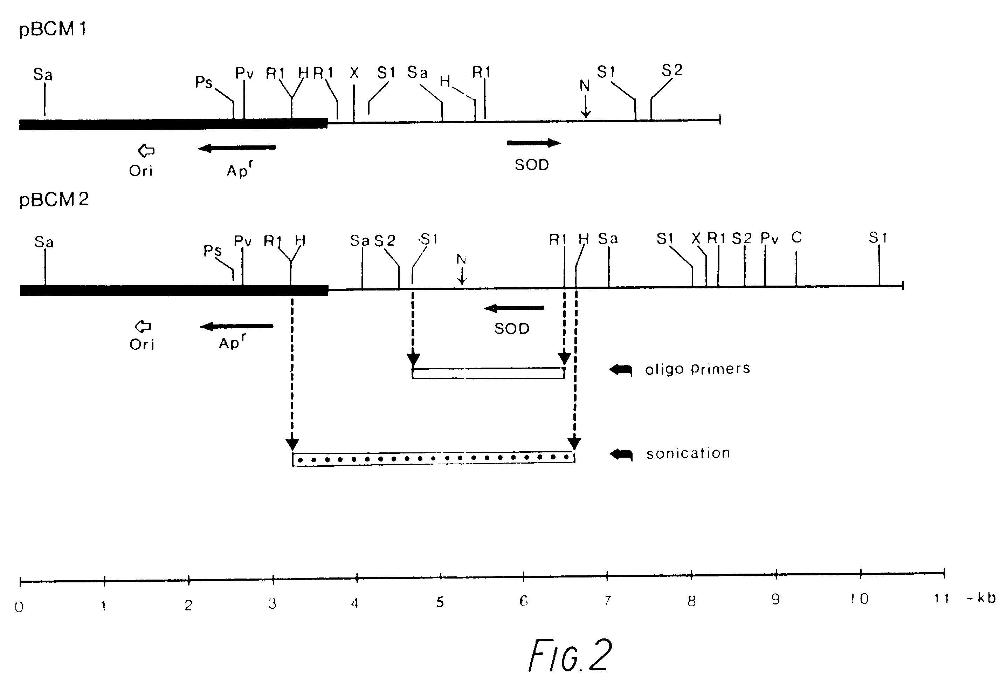

Having demonstrated the specificity of the oligonucleotide probe, a plasmid library of the B. stearothermophilus genome was constructed by ligating sized (approx. 8 kb), partially digested (Sau3a) chromosomal DNA with BamHI-cleaved pAT153 DNA. The resultant ligation mixtures were transformed into E.coli W5445 and transformants selected on L-agar containing ampicillin. Of the 6,000 ApR transformants obtained, 4,125 proved to be TcS. Upon analysis of the plasmids of 50 random representatives of the 4,125 presumptive recombinant clones, 46 were shown to contain inserts. Each ApR TcS recombinant clone was individually screened by in situ colony hybridisation, using the radiolabelled oligonucleotide as a probe. The probe was shown to hybridise strongly to two different recombinant clones. Plasmid DNA was isolated from each clone and designated pBCM1 and pBCM2. A restriction map of these two plasmids is illustrated in Fig. 2. These maps demonstrate that the insert of pBCM1 was 4.7 kb, while that of pBCM2 was 6.85 kb in size. Comparative analysis of DNA fragments generated by digestion (singularly or in double combinations) with various restriction enzymes indicated that the insert of pBCM2 entirely encompassed that of pBCM1, and furthermore, that the insert of pBCM1 was in the opposite orientation, relative to the vector, to that of pBCM2.

In order to localise the position of the sod structural gene within cloned DNA present in pBCM1 and pBCM2, each plasmid was restricted with various endonucleases and the resultant fragments subjected to Southern blot analysis. One of the smallest restriction fragment which hybridised to the oligo probe was shown to be a 1.6 kb EcoRISs tI fragment common to both plasmids.

Accordingly this fragment was gel purified from pBCM2 DNA and ligated to M13mp18 and M13mp19 similarly cleaved with EcoRI and SstI. The ligation mixes were transformed into E.coli TG1 and plated on 2XYT agar in H- top agar overlays containing XGal and IPTG. Recombinant plaques, identified by their colourless appearance, were utilised to prepare template DNA. Representative templates derived from each M13 vector were than subjected to nucleotide sequence analysis using universal primer. Translation of the DNA sequence obtained from both templates into amino acid sequence failed to yield an ORF encoding a polypeptide homologous to the published MnSOD sequence. This suggested that sod resided within the central portion of this fragment. Thereafter, the complete sequence of the insert was determined using two different strategies: (i) oligonucleotides specific to the sequence derived from the above two templates were synthesised and used to extend the previously determined sequence; (ii) the 3.0 kb HindIII fragment of pBCM2 which encompasses the cloned 1.6 kb EcoRI-SstI fragment was isolated by electroelution, circularised by self-ligation. fragmented by sonication, the staggered ends generated blunt-ended by treatment with T4 polymerase, and gel purified fragments of 500 to 1000 bp inserted into the SmaI site of M13mp8.

Template DNA was prepared from 100 of the recombinant clones obtained. The nucleotide sequence data obtained was assembled into one contiguous sequence using the computer software of DNASTAR Inc. The sequence illustrated in Figure 3 represents a 1294 bp portion of the sequence obtained which encompasses the sod structural gene, and was determined on both DNA strands.

Translation of the nucleotide sequence illustrated in Fig. 3 revealed the presence of an ORF of 615 bp beginning with an AUG codon (nt 387) and terminating with a UAA codon (nt 1001). The deduced polypeptide was 204 aa in length and, with the exception of the N-terminal Met, exhibited perfect conformity to the experimentally determined aa sequence of MnSOD (Brock and Walker, 1980).

A sequence beginning at 438 and ending at 488 exhibited near perfect complimentarity to the oligo probe utilised to identify the gene. The three positions at which mismatch occurred (nt 465, 477 and 483) all resulted in neutral G.T pairing, accounting for efficient binding of the oligo to B.stearothermophilus- derived DNA encoding sod. The translational initiation codon was preceded by a sequence (5'-CAAAAGGAGGAGA-3') exhibiting strong complimentarity to the 3'-termini of the Bacillus subtilis 16S rRNA (3'-UCUUUCCUCCACU-5'). A sequence exhibiting dyad symmetry occurs immediately 3' to the translational stop codon (nt 1007 to 1036), and probably represents a Rho-independent transcriptional terminator. The putative RNA stem-loop structure formed would have a DG of -22.2 kcal. A second putative ORF was identified 3' to sod, initiating with an AUG codon at nt 1133 and preceded by a sequence exhibiting reasonable complimentarity to the B.subtilis 16S rRNA. The encoded putative polypeptide exhibits no homology to any protein currently found in the PIR database. The sod structural gene exhibits a G+C content of 53.1%, and its codon usage is illustrated in Table 2.

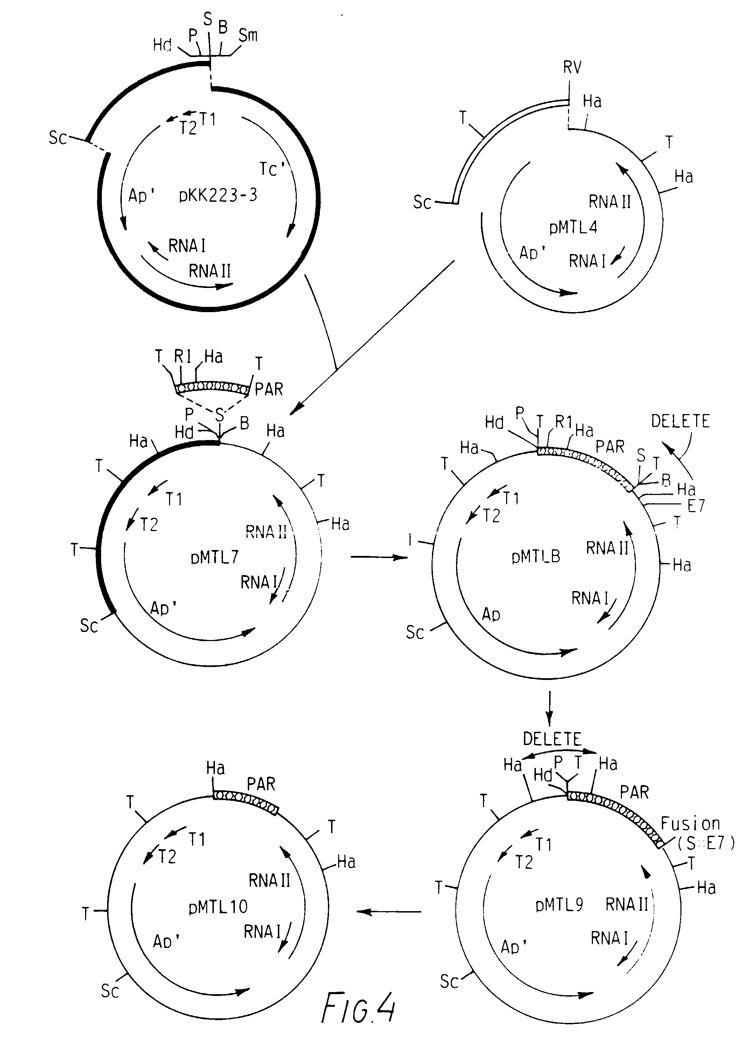

To elicit the overexpression of sod in E.coli use was made of the plasmids pMTL1003 and pMTL1013, part of a series of expression vectors recently constructed in this laboratory. Derived from pMTL4 (Chambers et al., 1988), pMTL1003 replicates from a mutant ColE1 replicon (600 copies per cell; Minton et al., 1988), encodes pUC8-derived bla and lacZ' (Messing and Vieria, 1982), and incorporates the pSC101 partition function (par; Miller et al., 1983), the E.coli rrnB double terminator (Brosius et al., 1981) and the pMTL20 polylinker cloning region (Chambers et al., 1988). Transcription of lacZ' is under the control of a synthetic trp promoter. pMTL1013 differs from pMTL1003 only in that the bla gene (ApR) has been replaced with the tet (TcR) gene. These expression vectors were derived in the following manner (see Fig. 4 for details).

The 5' end of the bla gene (ApR) was isolated, together with the double transcriptional termination signals (T1 and T2) of the rrnB operon, from the plasmid pKK223-3 as a 831 bp ScaI/SmaI fragment, and inserted between the EcoRV and ScaI sites of pMTL4, to give pMTL7. A 385 bp TaqI fragment carrying the pSC101 par stability determinant (Miller et al., 1983) was then inserted between the EcoRV and ScaI sites to yield pMTL8. The remaining manipulations were designed to both reduce the size and remove unwanted restriction sites from the final vector. pMTL8 was cleaved with SalI and Eco47, blunt-ended with S1 nuclease and self-ligated to give pMTL9. This 58 bp deletion removed the unique SalI, Eco47, and BamHI sites and a number of TaqI and HaeII sites. A 322 bp HaeII fragment was then deleted from pMTL9, reducing the number of HaeII and TaqI sites in the final vector, pMTL10, by one, and removing the unique PstI and HindIII sites. Although this deletion removed part of the pSC101 par fragment, pMTL10 was shown experimentally to exhibit 100% segregationally stability in cells grown in the absence of antibiotic selection. The final modification made to the basic vector backbone was to employ site-directed mutagenesis to introduce a unique EcoRV site between par and the ColE1 origin of replication.

This was achieved by first cloning the 530 kb TaqI fragment of pMTL10 encompassing this region into the AccI site of M13mp8. A mutagenic oligonucleotide was then employed to introduce the desired EcoRV restriction site using site-directed mutagenesis. The mutated TaqI fragment was then reisolated and ligated to the 1.44 kb and 430 kb TaqI fragments of pMTL10. The modified vector obtained was designated pMTL100.

In order to place the expression of a heterologous gene under the transcriptional control of an extraneous promoter it is necessary to insert the structural gene in the correct orientation adjacent to the appropriate transcriptional signals. Such manipulative procedures are enhanced by the facility for directional cloning and by the existence of a means of detecting the insertion of the foreign DNA.

One of the simplest systems, exemplified by the pUC and M13 series of vectors (Vieira and Messing, 1982) is that involving inactivation of the b-galactosidase a-peptide encoded by lacZ'. Vectors carrying a functional lacZ' confer a blue colouration to the colonies or plaques of appropriate E.coli hosts in the presence of the chromogenic substrate XGal. Inactivation of the gene (ie., by the insertion of heterologous DNA) results in the colourless (white) colonies/plaques. In the pUC and M13 vectors, the lacZ' gene is proceeded by its natural lac po promoter region.

In the vectors pMTL1003 and pMTL1013, we wished to retain the utility of the lacZ' selection system but replace the lac promoter with that of the trp promoter. To achieve this site-directed mutagenesis of M13mt120 DNA was used to remove a PvuII site within the 3' end of lacZ' (leaving the PvuII site 5' to the lac po unique) and to create a unique HpaI site 3' to the +1 of the lac po at the same time an NdeI site was created at the start of the lacZ' gene, such that the ATG of the NdeI recognition sequence (CATATG) corresponded to the AUG translational initiation codon of lacZ'.

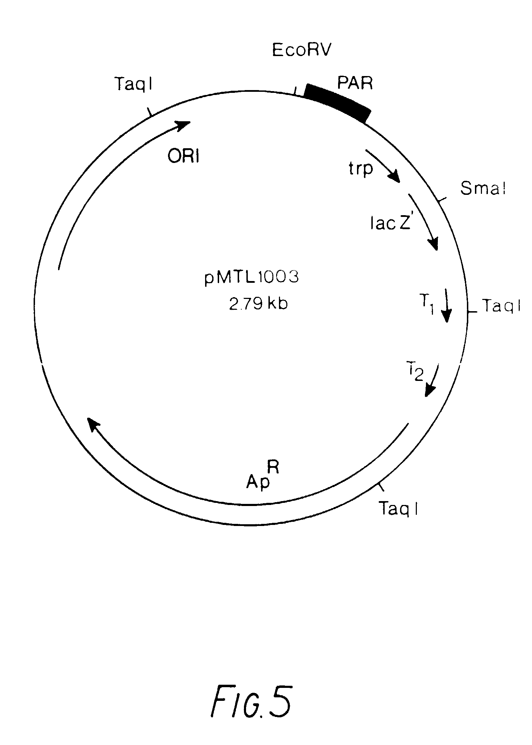

Although not relevant to the expression of SOD, its presence will aid in the future expression of other genes. eg., an NdeI site may be created at the equivalent position in any heterologous gene to be expressed, and then used to insert the gene at the NdeI site of the modified lacZ'. This places the AUG start codon of the gene to be expressed at the optimum distance from the Shine-Dalgarno of the lacZ' gene, maximising subsequent translational of RNA transcripts. A 830 bp HindIII-PstI restriction fragment carrying a synthetic E. coli trp promoter was isolated from plasmid pDR720 (Russell and Bennett 1982), blunt-ended by treatment with PolIk and inserted between the PvuII and HpaI sites of the modified M13mt120 vector. This manipulation effectively substituted the natural lac promoter with that of trp [NB. the same strategy may be employed to replace lac po with any other promoter element]. The trp promoter/lacZ'/polylinker region was then removed from the M13 vector as a 388 bp HaeII fragment and cloned into one of the two HaeII sites of pMTL100, in the indicated orientation (Fig. 5) to give pMTL1003.

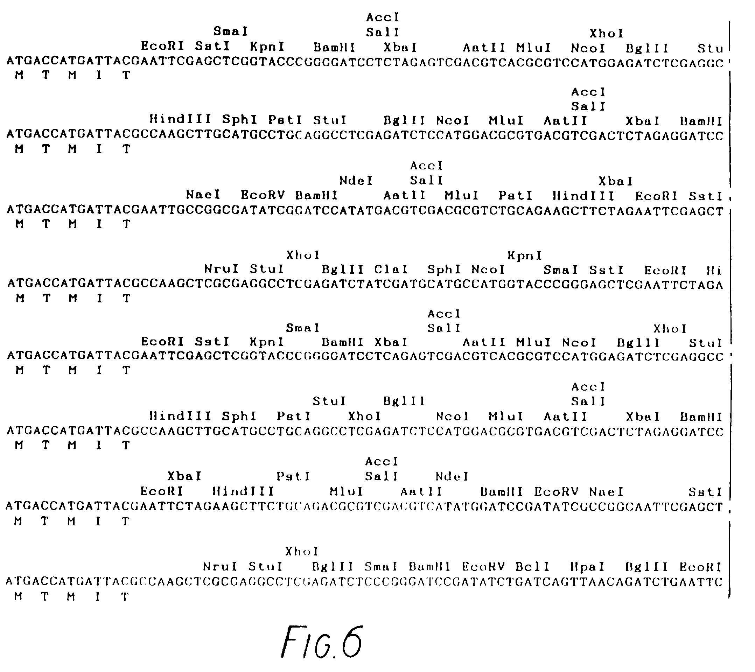

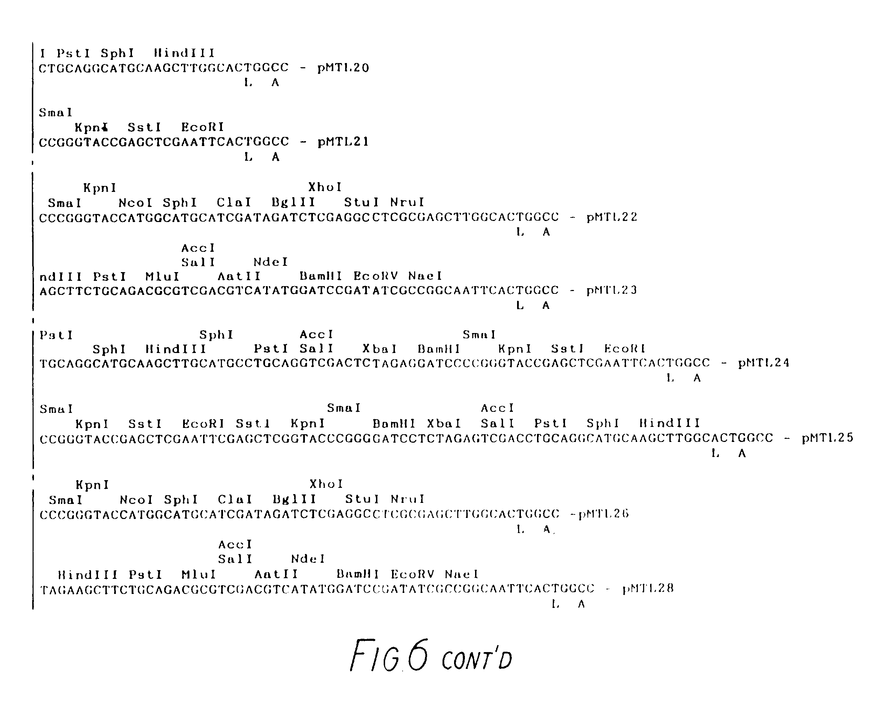

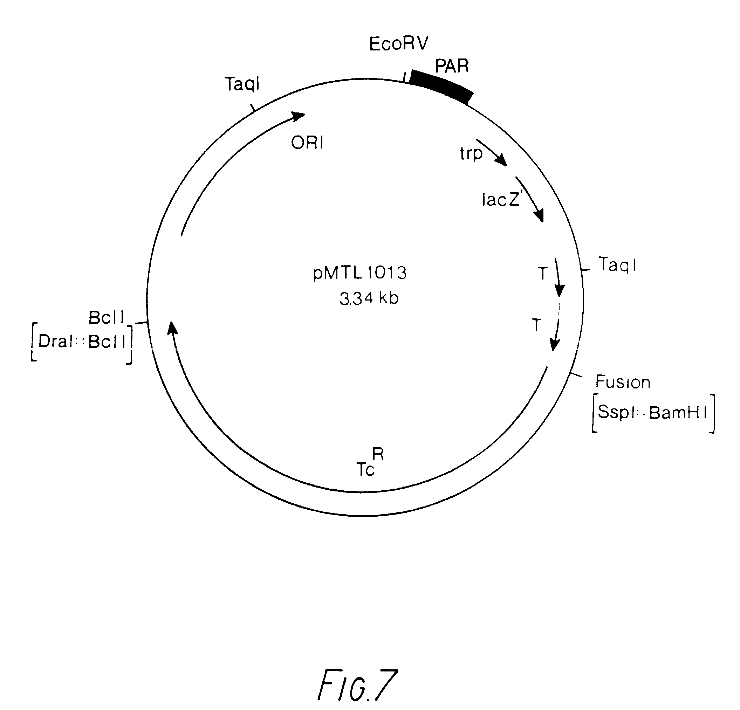

The vector pMTL1013 is analogous to pMTL1003, except that the bla gene has been replaced with the pBR322 tet gene. The tet gene was isolated from pBR322 as a 1.43 kb EcoRI-AvaI fragment (Balbas et al., 1986), blunt-ended with PolIk and inserted into the SmaI site of M13mp10. Site-directed mutagenesis was then employed to remove restriction enzyme sites for ClaI, HindIII, EcoRV, BamHI, SphI, and SalI. The respective nucleotide substitutions were: A to T, nt 27; T to A, nt 28; T to C, nt 187; C to T, nt 379; T to C, nt 565, and; C to T, nt 656 (nucleotide positions correspond to the pBR322 sequence, Balbas et al., 1986). The modified tet gene was then excised as a approx. 1.43 kb EcoRI-BamHI fragment inserted into the HpaI site of pMTL28 (see Fig. 6), re-isolated as a BamHI-BclI fragment and ligated to a 1.85 kb fragment of pMTL1003 generated by cleavage of pMTL1003 with SspI and DraI. The final plasmid obtained was designated pMTL1013 (Fig. 7).

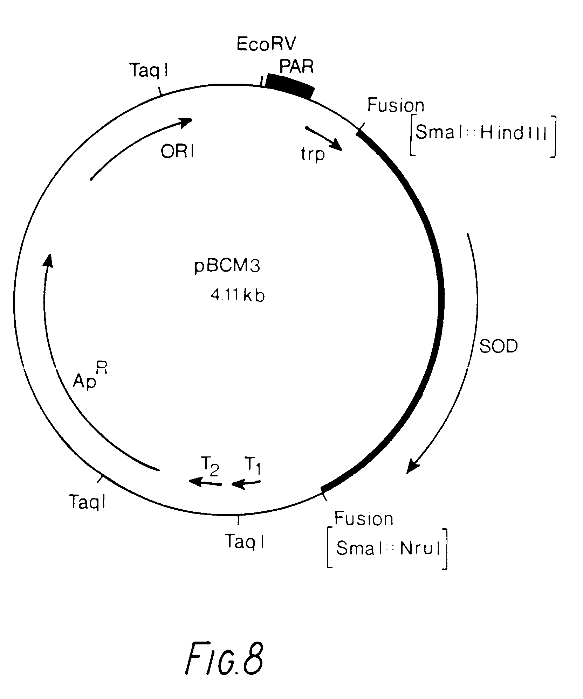

The sod gene was isolated from pBCM2 as a 1.3 kb NruI-HindIII fragment (Fig. 3), cloned between the SmaI and HindIII restriction sites of pUC9 and then re-excised as a similarly sized EcoRI-HindIII fragment. This fragment was then inserted, following blunt- ending by treatment with PolIk, into SmaI site of the pMTL1003. Two recombinant plasmids (pBCM3 and pBCM4), representing the two possible orientations of insertion of the cloned fragment, were chosen for further study. In the case of pBCM3 (Fig. 8), sod was orientated such that its expression could be enhanced by transcriptional read-through from the vector trp promoter. Two analogous plasmids pBCM5 (equivalent to pBCM3) and pBCM6 (equivalent to pBCM4) were generated by using pMTL1013 in place of pMTL1003

Cells harbouring pBCM3 and pBCM4 were grown in complex media (2XYT), supplemented with 100 lM MnSO₄, and transcription from the vector trp promoter induced in late exponential phase by the addition of indole acrylic acid (20 lg/ml). Cells were removed from the cultures at hourly intervals, disrupted by sonication and the SOD activity of the extract determined following removal of cell debris by centrifugation.

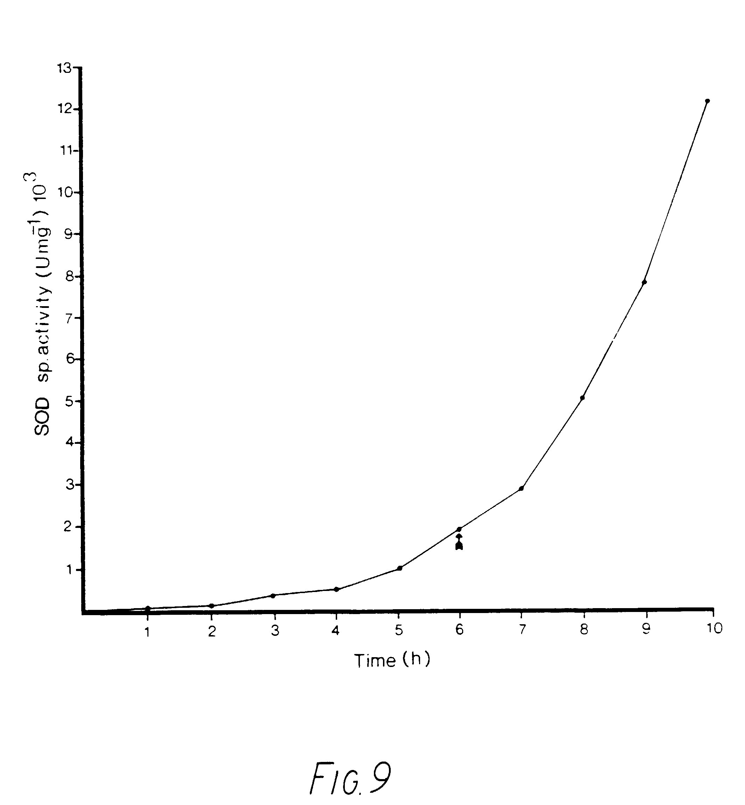

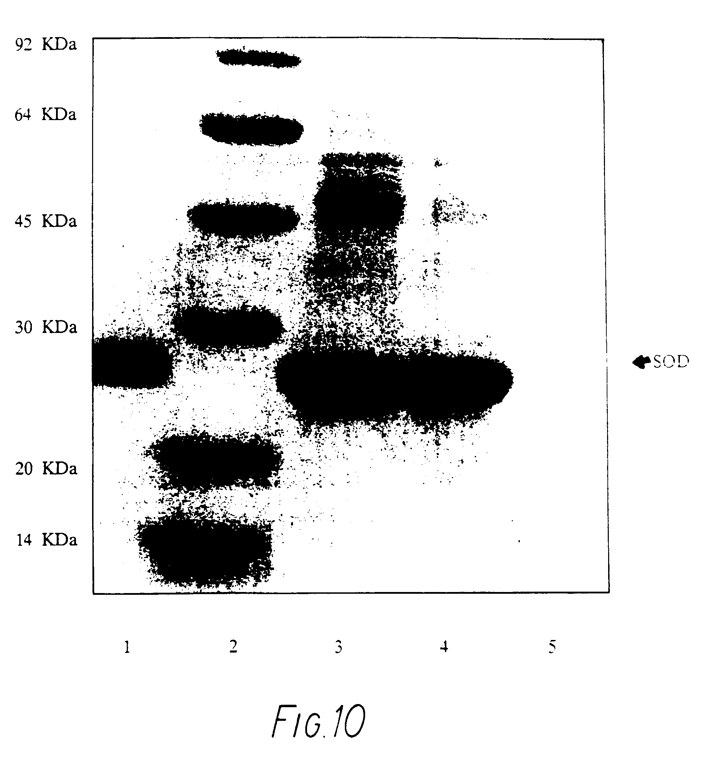

The maximum level of MnSOD produced by cells carrying pBCM3, 62,275 units per ml of culture (equivalent to 12,210 u/mg soluble protein), was attained after 10 h (Fig. 9). By reference to the specific activity of pure MnSOD (25,000 u/mg), this equated to 47% of the cells soluble protein. Confirmation of these levels was obtained by densiometric scanning of Coomassie blue stained gels following SDS-PAGE of total cell extracts (see Fig. 10).

That high expression was due to the vector trp promoter was indicated by the low level of SOD produced (10.9 units per ml of culture) by cells harbouring pBCM4. The surprising ability of E.coli to support high level of expression of the B.stearothermophilus sod gene is consistent with the observation that its encoding region makes little use of modulator codons (a single CGG and a GGA codon are used), exhibits a codon bias characteristic of highly expressed E.coli genes and is preceded by a near to consensus ribosome binding site.

The levels of sod expression directed by pBCM3 were examined in a range of E.coli hosts with varying degrees of native SOD activity (Table 3). In this case transcription from the trp promoter was induced late in their exponential phase following tryptophan depletion from the minmal salts medium described in the Examples. Inexplicably, hosts carrying a mutant sodB locus produced significantly lower levels of recombinant SOD than either a sodA or wt host.

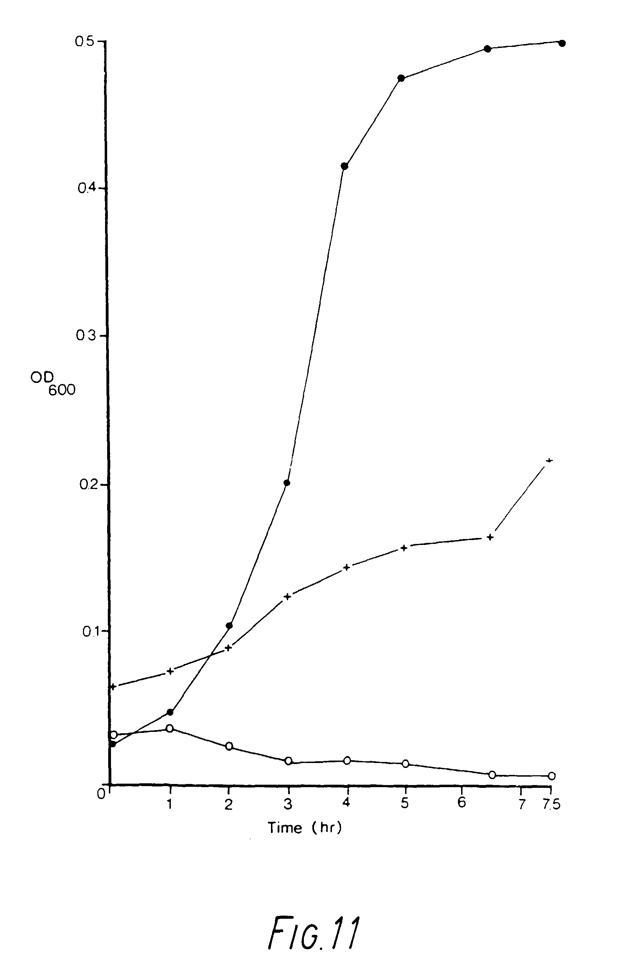

Previous studies have shown that sod mutants exhibit enhanced sensitivity to methyl viologen (Carlioz and Touati, 1986), a commercial weed killer known to generate superoxide free radicals in E.coli. It was therefore of interest to see whether the B. stearothermophilus enzyme was capable of complementing the enhanced sensitivity of the E.coli strain QC781 to methyl viologen. Strain QC781 with and without pBCM3 was therefore grown in the presence of 10-5 M methyl viologen and the effect on growth rate quantified. The results are illustrated in Fig. 11. Expression of recombinant SOD was seen to alleviate the growth inhibitory effect of the drug, but did not completely restore growth rates to those attained by QC781 in the absence of methyl viologen. This is in contrast to similar experiments undertaken with a cloned yeast MnSOD (Schrank et al., 1988).

The production of the MnSOD gene of B stearothermophilus and B. caldotenax will be described in more detail in the following examples. The bacterial strains and recombinant vectors utilised are listed in Table I.

### EXAMPLE 1

### (a) Media and culture conditions

B. stearothermophilus was grown at 58°C and pH of 7.0 with an air flow rate of 1 vvm in the following medium; sucrose (4%), yeast extract (5%), KH₂PO₄ (1%), MgSO₄.7H20 (0.054%). MnCl₂.4H₂O (0.003%), FeCl₃.H₂O (0.0014%), citric acid monohydrate (0.064%), polypropylene glycol P-2000 (0.01%). E.coli was routinely cultured in L-broth (1% tryptone, 0.5% yeast extract, 0.5% NaCl). Solidified medium (L-agar) consisted of L-broth with the addition of 2% (w/v) agar (Bacto-Difco). Antibiotic concentrations used for the maintenance and the selection of transformants were 50 lg/ml ampicillin, 15 lg/ml tetracycline, 30 lg/ml kanamycin and 5 lg/ml chloramphenicol. Repression of the trp promoter, when necessary, was obtained by the presence of an excess of tryptophan in the media (100 lg/ml). The medium used in the pilot scale production of recombinant SOD in E.coli contained glucose (1.4%), NH₄SO₄ (0.25%), KH₂PO₄ (0.3%), K₂HPO₄ (0.2%),Na.citrate (0.005%), yeast extract-Difco (0.5%), MgSO₄ (1%) and trace elements (1.0%). Stock solution of trace elements EDTA.Na₂ (0.5%), FeCl₃.6H₂O (0.05%), ZnO (0.005%), CuCl₂.H₂0 (0.001%), CoNO3.6H₂O (0.001%) and NH₄Mo₇O₂₄ (0.001%). The culture was grown at 37°C at a pH 7.0 +0.1 with an air flow rate of 1 vvm. Under these conditions exponential growth ceased after about 8 hours at which time the culture was harvested.

### (b) Purification of DNA

Plasmids were purified from cleared lysates prepared using a Brij-lysis procedure (Clewell et al., 1969) and subsequent caesium chloride-ethidium bromide density gradient centrifugation (Colman et al., 1978). A rapid, small scale plasmid purification technique (Holmes and Quigley, 1981) was also employed for screening purposes. Chromosomal DNA from the donor B.stearothermophilus was prepared essentially as described by Marmur (1961).

### (c) Restriction, ligation and transformation methods

Restriction endonucleases and DNA ligase were purchased from Bethesda Research Laboratories (BRL) and used in the buffers and under the conditions recommended by the supplier. Transformation of E.coli was essentially as described by Cohen et al. (1972).

### (d) Agarose gel electrophoresis

Digests were electrophoresed in 1% agarose slab gels on a standard horizontal system (BRL Model H4), employing Tris-borate-EDTA buffer. Electrophoresis of undigested DNA was at 125 V, 50 mA for 3 h, while digested DNA was electrophoresed at 15 V, 10 mA for 16 h. Fragment sizes were estimated by comparison with fragments of phage k DNA cut with both HindIII and EcoRI. Fragments were isolated from gels using electroelution (McDonnell et al., 1977).

### (e) Nucleotide sequencing

M13 bacteriophage clones were sequenced by the dideoxynucleotide method of Sanger et al (1977) using a modified version of bacteriophage T7 DNA polymerase, "sequenaseR" (Tabor and Richardson, 1987). Experimental conditions used were as stated by the supplier (USB Corp.). Sequencing of double-stranded plasmid DNA was by a modification of the Klenow polymerase- dideoxynucleotide method developed by Chen and Seeburg (1985). Experimental conditions used were as stated by the supplier (BCL).

### (f) Southern transfer of DNA

DNA restriction fragments were transferred from agarose gels to "zeta probe" nylon membrane by the method of Reed and Mann (1985). Gels were partially depurinated with 0.25 M HCL (15 min) prior to transfer in 0.4 M NaOH transfer solution. Transfer was carried out for 4-16 h by capillary elution prior to hybridisation.

### (g) In situ colony hybridisations

Bacterial colonies were screened for desired recombinant plasmids by in situ colony hybridisation as described by Grunstein and Hogness (1977), using nitrocellulose filter disks (Schleicher and Schull, 0.22 lm).

### (h) Radiolabelling of oligonucleotides

Oligonucleotide probes were end-labelled by the addition of [c-32P] dATP to the 5'- hydroxyl terminal with T4 polynucleotide kinase (Maxam and Gilbert, 1977). Unincorporated nucleotides were removed by chromatography through sephadex G-25 disposable columns as specified by the manufacturer (Pharmacia).

### (i) Hybridisation conditions

Hybridisations using the 5'-end-labelled 50 mer oligonucleotide probe were carried as described by Sambrook et al (1989) at a temperature of 55°C for 2 or more h. Filter washes were carried out at 45°C, several times of 5 min duration.

### (j) Site-directed mutagenesis

Coupled priming oligonucleotide -directed mutagenesis was carried out using the suppressor selection protocol of Carter et al (1985). Mutants were identified by the differential temperature hybridisation method described by Carter et al (1984) using radiolabelled oligonucleotide as probe.

### (k) Segregational stability

The segregational stability of plasmid vectors was analysed using continuous culture. Cells were grown in a LH500 series fermenter and control package in a 1 litre continuous culture vessel in a working volume of 600 ml. The growth medium employed was the simple salts medium of Tempest (1969). Cultures were maintained at 37°C, pH 7.0 and with an aeration rate of 1 vvm. Following inoculation, cultures were allowed to grow batchwise for 4 to 5 h before the flow of fresh medium was initiated. Samples were removed periodically and serially diluted onto isosensitest agar and colonies screened for plasmid encoded b- lactamase production.

### (l) Determination of superoxide dismutase activity

Bacteria were grown in 1 litre batch culture and 100 ml samples taken at various stages in the growth phase. Samples were cooled on ice, centrifuged 13,000 g for 120 minutes and resuspended and frozen in 5 ml 50mM Phosphate buffer (pH 7.8). The cells were disrupted using a MSE Ultrasonic Disintegrator (150 W) at medium frequency, amplitude 2, for three 30 second intervals on ice. Cell debris was removed by centrifugation at 10,000 g for 5 minutes. SOD activity was measured by monitoring the inhibition of reduction of ferric cytochrome C, as described by McCord and Fridovich (1979). Protein concentration was determined by the method of Bradford (1976). SOD activity was also visualised following PAGE, the gel was soaked in a solution of nitro- blue tetrazolium reagent before adding riboflavin. This procedure was fully described by Beauchamp and Fridovich (1971).

### (m) Small-scale fermentation of E.coli TG1 containing pBCM3

Although the level of expression of recombinant SOD obtained in batch culture was of a high order of magnitude, it was of interest to see whether high production rates could be translated to conditions more closely resembling those employed for commercial production of recombinant proteins. Accordingly, an 8 1 pilot-scale culture was carried out using the minimal salts medium described herein. The inoculum for the seed was provided by freshly transformed cells plated out onto L-agar supplemented with tryptophan (100 lg/ml) and ampicillin (100 lg/ml) for promoter repression and selection, respectively. The seed was provided by a 500 ml 2xLB culture supplemented with MnSO₄, ampicillin and tryptophan. The seed was grown at 37°C at 200 rpm for 7 h. Once inoculated the culture was allowed to go its full course before harvesting , relying on tryptophan starvation to switch on the trp promoter late in the cultures exponential phase of growth, when cell densities will be at their highest. Cells were harvested by centrifugation, and the cell paste bagged, flash frozen and stored at -80°C until extracted. The level of SOD expression obtained from the pilot scale cultures were consistent with those obtained in shake flask experiments. Following purification characterisation of the purified recombinant SOD identified the protein as a dimer, with each subunit having a molecular weight of approximately 21,000 dal and an isoelectric point of 5.5.

From the above results it can be seen that the gene (sod) encoding Bacillus stearothermophilus Mn-superoxide dismutase has been cloned in Escherichia coli and its entire nucleotide sequence determined. With the exception of the post-translationally cleaved N-terminal methionine residue, the predicted amino acid sequence exhibits 100% similarity to the previously determined amino acid sequence. The recombinant MnSOD was shown to be functionally active in E. coli, both in vitro and in vivo, and was expressed to 47% of the cells soluble protein by coupling its transcription to the E. coli trp promoter.

### EXAMPLE 2

### (a) Molecular cloning of the B. caldotenax sod gene

The oligonucleotide probe utilised to clone the B.caldotenax gene was radiolabelled and used in DNA/DNA hybridisation reactions against B. caldotenax YT1 genomic DNA cleaved with various restriction enzymes. Under the conditions employed (see below) the probe was shown to hybridise strongly to various discrete restriction fragments, including a 4.1 kb HindIII fragment. Accordingly, HindIII- cleaved genomic DNA of approximately this size was isolated from agaorose gels and ligated to HindIII cut pUC19 plasmid DNA. The resultant ligation mixtures were transformed into E.coli TG1 and transformants selected on L-agar containing ampicillin and XGal. A total of 1100 recombinants (white colonies) were individually screened by in situ colony hybridisation, using the radiolabelled oligonucleotide as a probe. The probe was shown to hybridise strongly to 4 different recombinant clones. Plasmid DNA from one of these clones was isolated and designated pBCM7.

### (b) Determination of the B. caldotenax sod nucleotide sequence

In order to localise the position of the sod structural gene within cloned DNA present in pBCM7, plasmid DNA was restricted with various endonucleases and the resultant fragments subjected to Southern blot analysis.

One of the smallest restriction fragments which hybridised to the oligo probe was shown to be a 1.9 kb AccI fragment. This fragment was gel purified from pBCM7 DNA circularised by self-ligation, fragmented by sonication, the staggered ends generated blunt-ended by treatment with T4 polymerase, and gel purified fragments of 500 to 1000 bp inserted into the SmaI site of M13mp8. Template DNA was prepared from 100 of the recombinant clones obtained. The nucleotide sequence data obtained was assembled into one contiguous sequence using the computer software of DNASTAR Inc. The sequence illustrated in Figure 12 represents a 780 bp portion of the sequence obtained which encompasses the sod structural gene, and was determined on both DNA strands.

Translation of the nucleotide sequence illustrated in Fig. 12 revealed the presence of an ORF of 615 bp beginning with an AUG codon (nt 30) and terminating with a UAA codon (nt 643). Over the region illustrated in Figure 12 there were 35 nucleotide differences to the equivalent region of the B.stearothermophilus genome. Of these, 21 occurred in the coding region of the gene, resulting in two amino acid differences between the two encoded polypeptides. Thus the BCMnSOD contains Glu and Ile amino acid residues at positions 103 and 188, respectively, whereas the BSMnSOD contains Asp and Val amino acids at the equivalent respective positions. As with the B.stearothermophilus gene, the translational initiation codon was preceded by a sequence (5'- CAAAAGGAGGAGA-3') exhibiting strong complimentarity to the 3'-termini of the Bacillus subtilis 16S rRNA (3'-UCUUUCCUCCACU-5'). Similarly, a sequence exhibiting dyad symmetry occurs immediately 3' to the translational stop codon (nt 654 to 677), and probably represents a Rho-independent transcriptional terminator. In this case, however, the putative RNA stem-loop structure formed would have a higher DG (-26.4 kcal) than the equivalent structure found downstream of the B.stearothermophilus gene ( DG -22.2 kcal) due to a single difference in the nucleotide sequence, viz., a 'T' to `C' substitution. The sod structural gene exhibits a G+C content of 52.8%, and its codon usage is illustrated in Table 2.

### (c) Overexpression of BCMnSOD

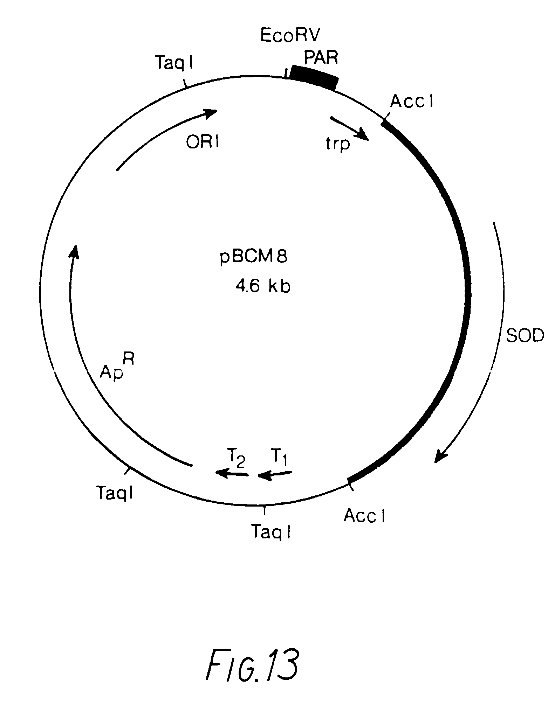

To elicit the high expression of the BCMnSOD gene, equivalent plasmids were constructed to those described above. In this case the 1.9 kb AccI fragment was inserted into the AccI site of pMTL1003, to give the recombinant plasmids pBCM8 (see Fig. 13) and pBCM9. In the case of pBCM8 (Fig. 13), sod was orientated such that its expression could be enhanced by transcriptional read-through from the vector trp promoter. Two analogous plasmids pBCM10 (equivalent to pBCM8) and pBCM11 (equivalent to pBCM9) were generated by using pMTL1013 in place of pMTL1003.

Cells harbouring pBCM8 and pBCM9 were grown in complex media (2XYT), supplemented with 100 µM MnSO₄, and transcription from the vector trp promoter induced in late exponential phase by the addition of indole acrylic acid (20 lg/ml). Cells were removed from the cultures at hourly intervals, disrupted by sonication and the SOD activity of the extract determined following removal of cell debris by centrifugation. The levels of expression attained mirrored those observed with the B. stearothermophilus recombinant clones. Thus the maximum level of MnSOD produced by cells carrying pBCM8, 90,710 units per ml of culture (equivalent to 9,913 u/mg soluble protein), was attained after 10 h (Fig. 9). By reference to the specific activity of pure MnSOD (25,000 u/mg), this equated to 40% of the cells soluble protein. Confirmation of these levels was obtained by densiometric scanning of Coomassie blue stained gels following SDS-PAGE of total cell extracts (see Fig. 10). That high expression was due to the vector trp promoter was indicated by the low level of SOD produced (8.9 units per ml of culture) by cells harbouring pBCM9. The ability of E.coli to support high level of expression of the B. caldotenax sod gene was consistent with the observation that its encoding region makes little use of modulator codons (a single CGG and a GGG codon are used), exhibits a codon bias characteristic of highly expressed E.coli genes (Grosjean and Friers, 1982), and is preceded by a near to consensus ribosome binding site.

### EXAMPLE 3

Purified, pyrogen-free BS MnSOD was produced from a culture of BS by the following procedure.

After harvesting, cells of BS were broken by high pressure homogenisation and the crude extract batch purified by fractional elution on DE-23 cellulose. The 0.4m fraction containing MnSOD was chromatographed sequentially as follows:
(i) DEAE-Sepharose by ion exchange gradient chromatography at pH 8.0
(ii) Hydroxylapatite chromatography using phosphate gradient at pH 6.8.

The 30% pure enzyme was depyrogenated and purified to homogeneity by ion exchange gradient chromotography on Q-sepharose at pH 7.5 and by gel filtration on sephaenyl S-200.

### PHARMACOLOGICAL TESTS

### (a) Serum half-life

The half life of BS MnSOD was assessed using a guinea-pig model. The effect of endogenous Cu/ZnSOD interference due to erythrocyte haemolysis was negated by the addition of 5mM cyanide to the assay system.

A half-life of approximately 6 hours was observed.

### (b) Antigenicity

No adverse antigenicity was observed in groups of guinea-pigs (n=12) receiving 1, 2 and 10 mg/kg body weight/6 hrs (4 animals/group) respectively via the intra-peritoneal route. Post-mortem investigation of animals sacrificed at 48 and 96 hours respectively (2 animals/dose/time) revealed no deleterious effect on internal organs and gross pathology was normal.

### (c) Protective effect during cardiac perfusion

A standard (Ringers) solution for cardiac perfusion was supplemented with 0.1 mg/l of BS MnSOD provided in vials containing 5mg enzyme, lOmg lactose and 5µmoles tris HCL.

Six mini-pigs were divided into two groups of 3 animals per group and subjected to procedure which mimicked human open-heart surgery. Specifically, the animals were maintained for 2h hours with clamped aortas while infusing with the test solutions.

At the end of the test period the aortic clamps were removed, normal blood supplies reconnected and standard methods used to restore normal sinus rhythm.

The animals were monitored in the post-operative period and the test animals (those infused with BS MnSOD-containing solutions) exhibited near normal cardiac function, and survived for one month at which time they were sacrificed for pathological examination. No signs of myocardial infarction or other abnormal cardiac tissue pathology was evident.

The animals in the control group exhibited cardiac malfunctions and were all dead after one week.

### APPENDIX

- Abbreviations:: aa, amino acid(s); Ap, ampicillin; BCMnSOD, Bacillus caldotenax MnSOD; bp, base pair(s); BSMnSOD, Bacillus stearothermophilus MnSOD; CuSOD, copper- containing SOD; dal, daltons; FeSOD, iron-containing SOD; kb, kilobase(s) or 1000 bp; kcal, kilocalories; lacZ', gene encoding the b-galactosidase a-peptide; MnSOD, manganese-containing SOD; nt, nucleotide(s); oligo(s), oligodeoxynucleotide(s); ORF, open reading frame; ORI, origin of replication; 02-, superoxide radical; PAGE, polyacrylamide gel electrophoresis; par, plasmid pSC101 partition function;po, promoter operator region; PolIk, Klenow (large) fragment of E.coli DNA polymerase I; R, resistance; S, sensitive; SDS, sodium dodecyl sulfate; SOD, superoxide dismutase; sod, gene encoding for SOD; Tc, Tetracycline; vvm, volumetric volume per minute; wt, wild type; XGal, 5-bromo-4-chloro-3-indolyl-b-D-galactoside; ZnSOD, zinc-containing SOD.

### FIGURE LEGENDS

### Figure 1. Oligonucleotide probe used to detect the B. stearothermophilus sod gene.

The indicated amino acids (single letter code, upper line) are residues 17 through 34 of the sequence determined by Brock and Walker (1980). The 50 mer oligonucleotide synthesised is labelled "probe", and was designed to complement the DNA strand encoding the targeted amino acid sequence. The actual sequence of the DNA encoding amino acids 17 through 34 is indicated above the oligonucleotide sequence (labelled "nt sequence"). Complementarity between the actual sequence and the probe is indicated by |, and neutral base pairing by a colon.

### Figure 2. Restriction enzyme map of pBCM1 and pBCM2.

Restriction enzyme sites are as indicated. DNA derived from pAT153 is represented by the thick line, the thin line representing the B. stearothermophilus-derived DNA insert. The positions and orientation of transcription of the sod (SOD) and bla (ApR) genes, and the ColEl origin of replication (ORI) are marked by appropriate arrows. The indicated 3.0 kb HindIII fragment and the 1.6 kb EcoRI-SstI fragment were isolated for sequencing puposes as outlined in the text. Restriction enzyme sites are: C, ClaI; H, HindIII; N, NruI; P, PvuI; R1, EcoRI; Sa, SalI; S1, SstI; S2, SstII, and; X, XhoI.

### Figure 3. Nucleotide sequence of the B. stearothermophilus gene encoding MnSOD

The illustrated region is a 1294 bp HindIII-NruI restriction fragment derived from pBCM2 (see Fig. 2). Of the two ORFs, ORF A corresponds to the sod gene and ORF B to the unidentified putative gene. Possible ribosome binding sites preceding both ORFs are underlined and labelled S.D. The region of dyad symmetry, which may correspond to the transcriptional terminator of sod, is indicated by facing arrows above the sequence.

### Figure 4. Construction of pMTL10.

Details on the individual steps involved in the construction of pMTL4 are given in the text. Restriction enzyme sites are :- Sc, ScaI; Hd, HindIII; P, PstI; S, SalI; B, BamHI; Sm, SmaI; RV, EcoRV; E7, Eco47; R1, EcoRI; T, TaqI, and; Ha. HaeII. Other plasmid specified elements are: the ColE1 replication specific transcripts, RNA I and RNA II; the pSC101 partition function, PAR; the E. coil trp promoter, trp; the b-galactosidase a-peptide, lacZ';, the E. coli rrnB operon transcriptional terminator signals, T1 and T2 and; the bla and tet genes, Ap and Tc.

### Figure 5. Restriction enzyme map of pMTL1003.

Plasmid pMTL1003 was derived from pMTL10 (see Fig. 4) by the insertion of a 388 bp HaeII fragment carrying trp/lacZ'/polylinker into the HaeII site of pMTL10 immediately adjacent to the pSC101 par element (see text for details). Labelled elements are: the ColE1 replication origin, ORI; the pSC101 partition function, PAR; the E. coli trp promoter, trp; the b-galactosidase a-peptide, lacZ'; the ampicillin resistance gene (bla), ApR, and; the E. coli rrnB operon transcriptional terminator signals, T1 and T2.

### Figure 6. Nucleotide sequence of the polylinker cloning region of pMTL28

The indicated polylinker regions correspond to those available in the pMTL20 cloning vector series (Chambers et al., 1988). pMTL28 was derived by chemically synthesising the appropriate oligos (5'-TCGAGATCTCCCGGGATCCGATATCTGATCAGTTAACAG- ATCTG-3' and 5'-AATTCAGATCTGTTAACTGATCAGATATCGGATCCCGG- GAGATC-3' ), annealing them and inserting them between the XhoI and EcoRI sites of pMTL23.

### Figure 7. Restriction enzyme map of pMTL1013

Plasmid pMTL1013 was constructed from pMTL1003 by substituting the bla gene with the tet gene (see text for details). Labelled elements are: the ColE1 replication origin, ORI; the pSC101 partition function, PAR; the E. coli trp promoter, trp; the b-galactosidase a- peptide, lacZ'; the tetracycline resistance gene (tet), TcR, and; the E. coli rrnB operon transcriptional terminator signals, T1 and T2.

### Figure 8. Restriction enzyme map of pBCM3

Plasmid pBCM3 was constructed by isolating the sod gene pBCM2 as a 1.3 kb NruI-HindIII fragment (Fig. 3), cloning it between the SmaI and HindIII restriction sites of pUC9 and then re-excised as a similarly sized EcoRI-HindIII fragment. This fragment was then inserted, following blunt-ending by treatment with PolIk, into SmaI site of the pMTL1003, such that transcriptional readthrough of sod could occur from the vector trp promoter. The B.stearothermopilus-derived DNA insert is represented by the thick line. Other features are: ColE1 origin of replication, ORI; pSC101 partition function, PAR; trp promoter, trp; rrnB transcriptional terminators, T1 and T2; ampicillin resistance marker and; the B. stearothermophilus MnSOD gene, SOD.

### Figure 9. Production of recombinant MnSOD in Escherichia coli carrying pBCM3

Cells harbouring pBCM3 were grown in complex media (2XYT), supplemented with 100 1M MnSO₄, and transcription from the vector trp promoter induced in late exponential phase (indicated by an arrow) by the addition of indole acrylic acid (20 lg/ml). Cells were removed from the cultures at hourly intervals, disrupted by sonication and the SOD activity of the extract determined following removal of cell debris by centrifugation.

### Figure 10. SDS-PAGE of total cell extracts of TG1 cells carrying pBCM3

Total cell extracts were derived from the 10 h sample of the experiment oultined in Fig. 9, and subjected to SDS-PAGE. Lane 1, purified B. stearothermophilus MnSOD; lane 2, molecular weight markers, and; lane 3, soluble cell lysate of TG1 carrying pBCM3.

### Figure 11. The ability of pBCM3 to complement an E.coli sodA mutant.

Strain QC781 was grown in the presence of 10-5 methyl viologen, either containing (+) or not containing (o) plasmid pBCM3. A growth curve of plasmid-free QC781 ( ) is also included for comparative purposes.

### Figure 12. Nucleotide sequence of the B. caldotenax gene encoding BCMnSOD

The illustrated region is a 780 bp portion of the 1.9 kb AccI fragment carried by pBCM8. Nucleotide sequence differences which occur in the B. stearothermophilus sequence are shown above the sequence in lower case (the `-' indicates the absence of a nucleotide in the B. stearothermophilus DNA). The two amino acid differences between BSMnSOD and BCMnSOD are illustrated by including the BSMnSOD amino acids below the BCMnSOD sequence at the appropriate positions (103, Asp instead of Glu; 188 Val in place of Ile). The ribosome binding site preceding the sod gene is underlined and labelled S.D. The region of dyad symmetry, which may correspond to the transcriptional terminator of sod, is indicated by facing arrows above the sequence.

### Figure 13. Restriction enzyme map of pBCM8

Plasmid pBCM8 was constructed by isolating the B. caldotenax sod gene from pBCM7 as a 1.9 kb AccI-fragment and cloning it into the AccI restriction site of the pMTL1003, such that transcriptional readthrough of sod could occur from the vector trp promoter. The B. caldotenax-derived DNA insert is represented by the thick line. Other features are: ColE1 origin of replication, ORI; pSC101 partition function, PAR; trp promoter, trp; rrnB transcriptional terminators, T1 and T2; ampicillin resistance marker and; the B. caldotenax MnSOD gene, SOD.

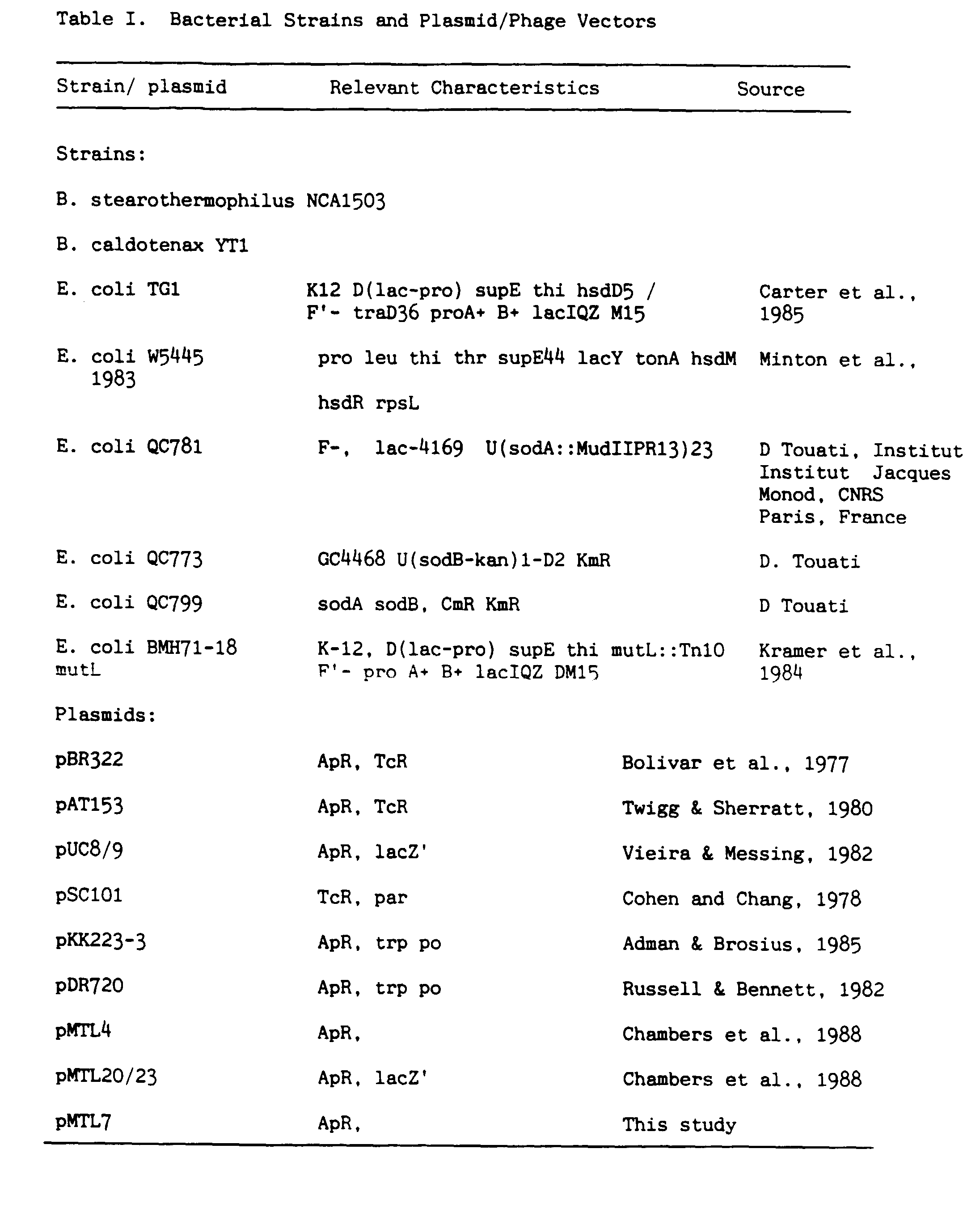

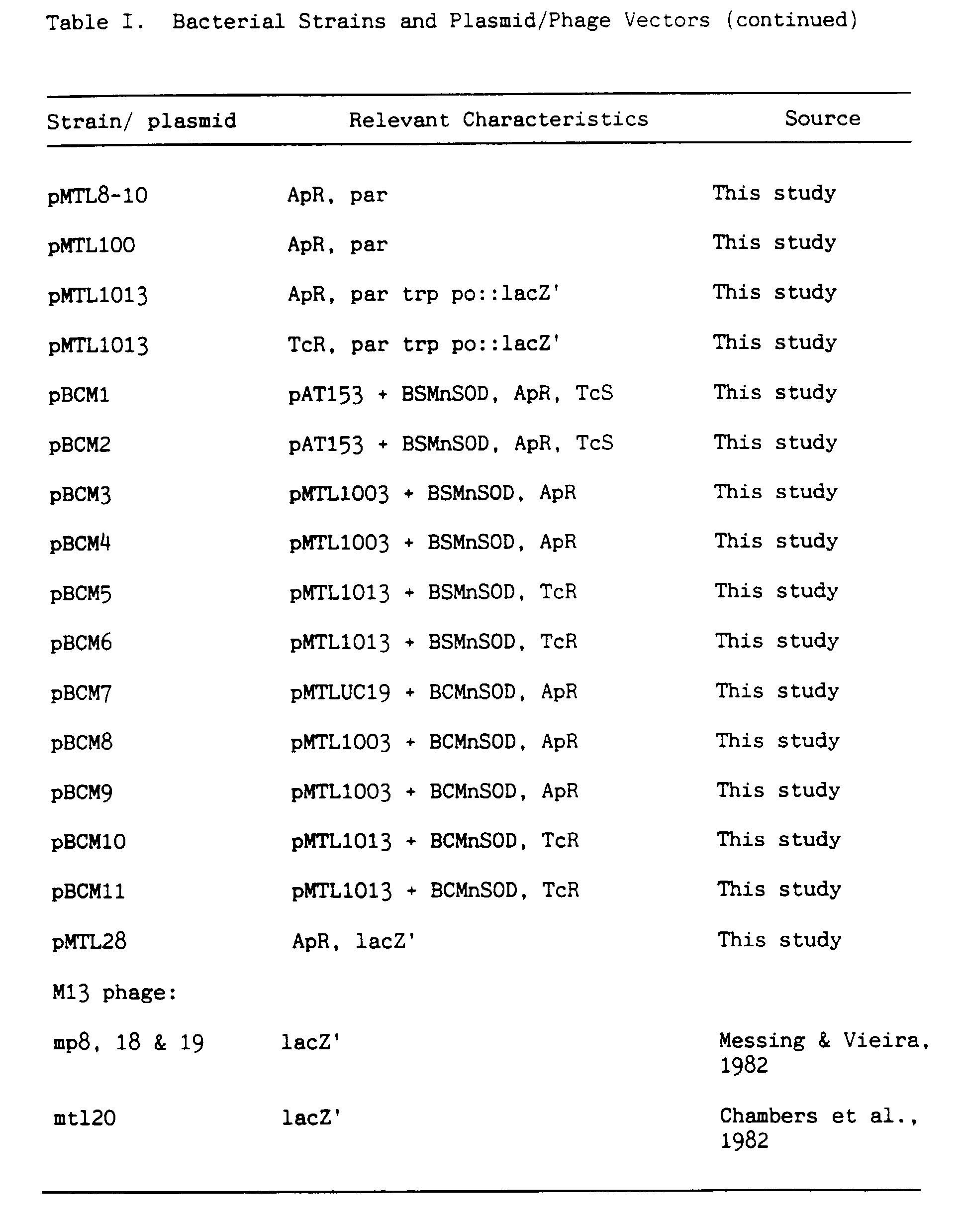

**Table 2.**

| Codon usage of the BSMnSOD and BCMnSOD genes | | | | | | | | | | | |
|---|---|---|---|---|---|---|---|---|---|---|---|
| | BS | BC | | BS | BC | | BS | BC | | BS | BC |
| UUU Phe | 2 | 3 | UCU | 0 | 1 | UAU Tyr | 2 | 2 | UGU Cys | 0 | 0 |
| UUC | 6 | 5 | UCC Ser | 1 | 0 | UAC | 6 | 6 | UGC | 0 | 0 |
| UUA Leu | 0 | 0 | UCA | 0 | 0 | UAA Ter | 1 | 1 | UGA Ter | 0 | 0 |
| UUG | 8 | 9 | UCG | 5 | 5 | UAG | 0 | 0 | UGG Trp | 6 | 6 |
| | | | | | | | | | | | |
| CUU | 4 | 5 | CCU | 0 | 0 | CAU His | 4 | 4 | CGU | 2 | 2 |
| CUC Leu | 3 | 3 | CCC Pro | 0 | 0 | CAC | 5 | 5 | CGC Arg | 3 | 3 |
| CUA | 0 | 0 | CCA | 3 | 3 | CAA Gln | 3 | 3 | CGA | 0 | 0 |
| CUG | 4 | 2 | CCG | 10 | 10 | CAG | 0 | 0 | CGG | 1 | 1 |
| | | | | | | | | | | | |
| AUU | 5 | 7 | ACU | 0 | 0 | AAU Asn | 4 | 4 | AGU Ser | 0 | 0 |
| AUC Ile | 4 | 3 | ACC Thr | 1 | 1 | AAC | 13 | 13 | AGC | 5 | 5 |
| AUA | 0 | 0 | ACA | 3 | 2 | AAA Lys | 11 | 10 | AGA Arg | 0 | 0 |
| AUG Met | 3 | 3 | ACG | 7 | 8 | AAG | 1 | 2 | AGG | 0 | 0 |
| | | | | | | | | | | | |
| GUU | 3 | 2 | GCU | 2 | 1 | GAU Asp | 3 | 2 | GGU | 3 | 4 |
| GUC Val | 2 | 2 | GCC Ala | 4 | 3 | GAC | 5 | 5 | GGC Gly | 11 | 10 |
| GUA | 0 | 0 | GCA | 5 | 5 | GAA Glu | 12 | 12 | GGA | 1 | 0 |
| GUG | 3 | 3 | GCG | 9 | 11 | GAG | 6 | 7 | GGG | 0 | 1 |
| Ter - corresponds to translational termination codon. BC - corresponds to the B. caldotenax gene. BS - corresponds to the B. stearothermophilus gene. embolden codons correspond to those codons recognised as modulators of translation in E. coli. | | | | | | | | | | | |

**Table 3.**

| Levels of Expression of native and recombinant SOD in E. coli. | | | |
|---|---|---|---|
| Host | Phenotypea | Plasmid | SOD specificb activity (U/mg) |
| TG1 | A+, B+ | - | 55.5 |
| QC781 | A-, B+ | - | 36 |
| QC773 | A+, B- | - | 1.9 |
| QC799 | A-, B- | - | 1.6 |
| | | | |
| TG1 | A+, B+ | pBCM3 | 12,000 |
| QC781 | A-, B+ | " | 12,000 |
| QC773 | A+, B- | " | 5,000 |
| QC799 | A-, B- | " | 4,000 |
| | | | |
| TG1 | A+, B+ | pBCM8 | 9,913 |
| QC781 | A-, B+ | " | 3,650 |
| QC773 | A+, B- | " | 2,581 |
| QC799 | A-, B- | " | 1,944 |
| a - Phenotypes A and B refer to the sodA and sodB gene, respectively, + or - indicating whether the gene is functional (+) or defective (-). b - The levels of sod expression directed by pBCM3/8 in a range of E.coli hosts, exhibiting varying degrees of native SOD activity, were estimated by assaying SOD levels in cell extracts (Table 3). In this case transcription from the trp promoter was induced late in their exponential phase following tryptophan depletion from the media (2XYT). | | | |

## Claims

1. A pharmaceutical composition for use in the prophylaxis and/or treatment of pathological conditions resulting from the presence of superoxide radicals, comprising an MnSOD enzyme and a pharmaceutically acceptable excipient, characterised in that the MnSOD enzyme (i) is in native form, (ii) catalyses the dismutation of superoxide radicals, and (iii) has (a) the amino acid sequence of *Bacillus Stearothermophilus* (BS) or *Bacillus Caldotenax* (BC) MnSOD or (b) a variant sequence that differs from the sequences in (a) by from 1 to 20 amino acid insertions, deletions and/or substitutions.

2. A pharmaceutical composition according to Claim 1 wherein the MnSOD enzyme in native form is essentially unmodified, chemically, compared to the MnSOD enzyme present in a culture of a MnSOD-producing BS or BC microorganism.

3. A pharmaceutical composition according to Claim 1 or Claim 2 which is substantially free of pyrogens consisting of macromolecular substances native to *B stearothermophilus* or *B caldotenax.*

4. A pharmaceutical composition according to any preceding claim wherein said MnSOD enzyme is produced by culturing a transformed microorganism being of a species other than *B stearothermophilus* or *B caldotenax.*

5. A pharmaceutical composition according to any preceding claim wherein said MnSOD enzyme has an amino acid sequence selected from
(i) the amino acid sequence depicted in Figure 12 for BC MnSOD,
(ii) the amino acid sequence depicted in Figure 3 for BS MnSOD, and
(iii) amino acid sequences which differ from the sequences (i) and (ii) by from 1 to 10 amino acid insertions, deletions and/or substitutions.

6. A pharmaceutical composition according to Claim 5 wherein said amino acid sequences (iii) differ from the sequences (i) and (ii) by from 1 to 5 amino acid insertions, deletions and/or substitutions.

7. A pharmaceutical composition according to any preceding claim in the form of (a) an injectable solution, or (b) a solution suitable for perfusing tissues during surgical or transplantation procedures.

8. A pharmaceutical composition according to Claim 7 containing from 0.001 to 1.0 mg/l of said MnSOD enzyme.

9. A pharmaceutical composition according to Claim 8 containing from 0.05 to 0.5 mg/l of said MnSOD enzyme.

10. A process for producing a pharmaceutical composition which comprises the steps of (a) culturing an MnSOD enzyme-producing microorganism so as to produce an MnSOD enzyme-containing culture, (b) isolating MnSOD enzyme from the culture, (c) purifying the isolated MnSOD enzyme so as to produce purified enzyme which is essentially unmodified, chemically, compared to the MnSOD enzyme present in the MnSOD-containing culture produced in step (a), and (d) mixing the chemically unmodified MnSOD enzyme with a pharmaceutically acceptable excipient, characterised in that said MnSOD enzyme (i) is in native form, (ii) catalyses the dismutation of superoxide radicals, and (iii) has (a) the amino acid sequence of *Bacillus Stearothermophilus (BS)* or *Bacillus Caldotenax* (BC) MnSOD or (b) a variant sequence that differs form the sequences in (a) by from 1 to 20 amino acid insertions, deletions and/or substitutions.

11. A process according to Claim 10 wherein the microorganism is a transformed microorganism of a species other than *B stearothermophilus* or *B caldotenax.*

12. The use of an MnSOD enzyme in the manufacture of a pharmaceutical composition for the prophylaxis and/or treatment of pathological conditions resulting from the presence of superoxide radicals, characterised in that the MnSOD enzyme (i) is in native form, (ii) catalyses the dismutation of superoxide radicals, and (iii) has (a) the amino acid sequence of *Bacillus Stearothermophilus* (BS) or *Bacillus Caldotenax* (BC) MnSOD or (b) a variant sequence that differs form the sequences in (a) by from 1 to 20 amino acid insertions, deletions and/or substitutions.

13. The use of an MnSOD enzyme in the manufacture of an infusing solution for organs undergoing surgery or transplantation, especially such organs which are isolated from normal blood supply, characterised in that the MnSOD enzyme (i) is in native form, (ii) catalyses the dismutation of superoxide radicals, and (iii) has (a) the amino acid sequence of *Bacillus Stearothermophilus* (BS) or *Bacillus Caldotenax* (BC) MnSOD or (b) a variant sequence that differs form the sequences in (a) by from 1 to 20 amino acid insertions, deletions and/or substitutions.

14. An MnSOD enzyme being in substantially pure form and having the amino acid sequence, or essentially the amino acid sequence, of *B caldotenax,* said amino acid sequence being selected from
(i) the amino acid sequence depicted in Figure 12,
(ii) amino acid sequences which differ from the sequence (i) by from 1 to 30 amino acid insertions, deletions and/or substitutions, with the proviso that said amino acid sequences (ii) have Glu in the location marked 103 and/or have lle in the location marked 188.

15. A recombinant DNA molecule comprising a DNA sequence coding for an MnSOD enzyme as defined in Claim 14, said recombinant DNA molecule preferably being selected from (a) the DNA coding sequence depicted in Figure 12 and (b) DNA sequences which are degenerate according to the genetic code to said sequence.

16. A process for producing an MnSOD enzyme which comprises culturing a transformed strain of *E.coli* containing a recombinant plasmid comprising at least one structural gene coding for an MnSOD enzyme operatively linked to a promoter, characterised in that said promoter is the native *trp* promoter of *E.coli.*

17. A process according to Claim 16 wherein said promoter has a base sequence comprising the sequence TTGACA at the 5' end and the sequence TTAACT at the 3' end.

18. A process according to Claim 16 wherein said promoter has a base sequence comprising the sequence TCAATT at the 5' end and the sequence ACAGTT at the 3' end.

19. A process according to any of Claims 17 to 18 wherein said promoter has the following intervening sequence located between said 3' and 5' sequences:

ATTAATCATCGAACTAG

20. A process according to Claim 16 wherein the promoter has the following base sequence:

TTGACAATTAATCATCGAACTAGTTAACT

## Patentansprüche

1. Pharmazeutische Zubereitung zur Verwendung bei der Prophylaxe und/oder Behandlung pathologischer Zustände aufgrund der Anwesenheit von Superoxidradikalen, welche ein MnSOD-Enzym und einen pharmazeutisch verträglichen Hilfsstoff enthält, dadurch gekennzeichnet, daß das MnSOD-Enzym (i) in nativer Form vorliegt, (ii) die Dismutation von Superoxidradikalen katalysiert und (iii) (a) entweder die Aminosäuresequenz von *Bacillus stearothermophilus* (BS) oder *Bacillus caldotenax* (BC) MnSOD oder (b) eine abweichende Sequenz aufweist, die sich von den Sequenzen in (a) durch 1 bis 20 Aminosäureinsertionen, -deletionen und/oder -substitutionen unterscheidet.

2. Pharmazeutische Zubereitung nach Anspruch 1, bei der das MnSOD-Enzym in nativer Form im Vergleich zum in einer Kultur eines MnSOD-erzeugenden BS- oder BC-Mikroorganismus vorhandenen MOD-SOD-Enzym im wesentlichen chemisch unmodifiziert ist.

3. Pharmazeutische Zubereitung nach Anspruch 1 oder 2, die im wesentlichen frei von aus *B. stearothermophilus* oder *B. caldotenax* von Natur aus eigenen makromolekularen Substanzen bestehenden Pyrogenen ist.

4. Pharmazeutische Zubereitung nach einem der vorstehenden Ansprüche, in der das MnSOD-Enzym durch Kultivieren eines transformierten Mikroorganismus aus einer anderen Spezies als B. *stearothermophilus* oder B. *caldotenax* hergestellt wird.

5. Pharmazeutische Zubereitung nach einem der vorstehenden Ansprüche, in der das MnSOD-Enzym eine aus
( i) der in Fig. 12 für BC MnSOD gezeigten Aminosäuresequenz,
( ii) der in Fig. 3 für BS MnSOD gezeigten Aminosäuresequenz und
(iii) Aminosäuresequenzen, die sich durch 1 bis 10 Aminosäureinsertionen, -deletionen und/oder -substitutionen von den Sequenzen (i) und (ii) unterscheiden
ausgewählte Aminosäuresequenz aufweist.

6. Pharmazeutische Zubereitung nach Anspruch 5, in der sich die Aminosäuresequenzen (iii) durch 1 bis 5 Aminosäureinsertionen, -deletionen und/oder -substitutionen von den Sequenzen (i) und (ii) unterscheiden.

7. Pharmazeutische Zubereitung nach einem der vorstehenden Ansprüche in Form (a) einer injizierbaren Lösung oder (b) einer Lösung, die sich zur Perfusion von Geweben während chirurgischer Eingriffe oder Transplantationen eignet.

8. Pharmazeutische Zubereitung nach Anspruch 7, die 0,001 bis 1,0 mg/l des MnSOD-Enzyms enthält.

9. Pharmazeutische Zubereitung nach Anspruch 8, die 0,05 bis 0,5 mg/l des MnSOD-Enzyms enthält.

10. Verfahren zur Herstellung einer pharmazeutischen Zubereitung mit folgenden Schritten:
(a) Kultivieren eines MnSOD-Enzym erzeugenden Mikroorganismus, um eine MnSOD-Enzym enthaltende Kultur herzustellen;
(b) Isolieren des MnSOD-Enzyms aus der Kultur;
(c) Reinigen des isolierten MnSOD-Enzyms zur Herstellung von gereinigtem Enzym, das im Vergleich zu dem in der in Schritt (a) hergestellten MnSOD enthaltenden Kultur vorhandenen MnSOD-Enzym chemisch im wesentlichen unmodifiziert ist, und
(d) Vermischen des chemisch unmodifizierten MnSOD-Enzyms mit einem pharmazeutisch verträglichen Hilfsstoff,
dadurch gekennzeichnet, daß das MnSOD-Enzym
( i) in nativer Form vorliegt,
( ii) die Dismutation von Superoxidradikalen katalysiert und
(iii) (a) entweder die Aminosäuresequenz von *Bacillus stearothermophilus* (BS) oder *Bacillus caldotenax* (BC) MnSOD oder (b) eine abweichende Sequenz aufweist, die sich von den Sequenzen in (a) durch 1 bis 20 Aminosäureinsertionen, -deletionen und/oder -substitutionen unterscheidet.

11. Verfahren nach Anspruch 10, bei dem der Mikroorganismus ein transformierter Mikroorganismus einer anderen Spezies als *B. stearothermophilus* oder *B. caldotenax* ist.

12. Verwendung eines MnSOD-Enzyms bei der Herstellung einer pharmazeutischen Zubereitung für die Prophylaxe und/oder Behandlung pathologischer Zustände aufgrund der Anwesenheit von Superoxidradikalen, dadurch gekennzeichnet, daß das MnSOD-Enzym (i) in nativer Form vorliegt, (ii) die Dismutation von Superoxidradikalen katalysiert und (iii) (a) entweder die Aminosäuresequenz von *Bacillus stearothermophilus* (BS) oder *Bacillus caldotenax* (BC) MnSOD oder (b) eine abweichende Sequenz aufweist, die sich von den Sequenzen in (a) durch 1 bis 20 Aminosäureinsertionen, -deletionen und/oder -substitutionen unterscheidet.

13. Verwendung eines MnSOD-Enzyms bei der Herstellung einer Infusionslösung für Organe, die operiert oder transplantiert werden, besonders solcher Organe, die von der normalen Blutzufuhr abgeschnitten sind, dadurch gekennzeichnet, daß das MnSOD-Enzym (i) in nativer Form vorliegt, (ii) die Dismutation von Superoxidradikalen katalysiert und (iii) (a) entweder die Aminosäuresequenz von *Bacillus stearothermophilus* (BS) oder *Bacillus* caldotenax (BC) MnSOD oder (b) eine abweichende Sequenz aufweist, die sich von den Sequenzen in (a) durch 1 bis 20 Aminosäureinsertionen, -deletionen und/oder -substitutionen unterscheidet.

14. MnSOD-Enzym, das im wesentlichen in reiner Form vorliegt und die Aminosäuresequenz oder im wesentlichen die Aminosäuresequenz von *B. caldotenax* aufweist, wobei diese Aminosäuresequenz ausgewählt ist aus
( i) der in Fig. 12 gezeigten Aminosäuresequenz,
(ii) Aminosäuresequenzen, die sich von der Sequenz (i) durch 1 bis 30 Aminosäureinsertionen, -deletionen und/oder -substitutionen unterscheiden mit der Maßgabe, daß die Aminosäuresequenzen (ii) Glu an der mit 103 bezeichneten Stelle und/oder Ile an der mit 188 bezeichneten Stelle aufweisen.

15. Rekombinantes DNA-Molekül, das eine DNA-Sequenz aufweist, die ein wie in Anspruch 14 definiertes MnSOD-Enzym codiert, wobei das rekombinante DNA-Molekül vorzugsweise aus (a) der in Fig. 12 gezeigten DNA-Codierungssequenz und (b) DNA-Sequenzen ausgewählt ist, die nach dem genetischen Code dieser Sequenz degeneriert sind.

16. Verfahren zur Herstellung eines MnSOD-Enzyms, bei dem man einen transformierten Stamm E. *coli* mit einem rekombinanten Plasmid, das mindestens ein Strukturgen aufweist, das für ein operativ mit einem Promotor verknüpftes MnSOD-Enzym codiert, dadurch gekennzeichnet, daß der Promotor der native trp-Promotor von E. *coli* ist.

17. Verfahren nach Anspruch 16, bei dem der Promotor eine Basensequenz hat, die die Sequenz TTGACA am 5'-Ende und die Sequenz TTAACT am 3'-Ende aufweist.

18. Verfahren nach Anspruch 16, bei dem der Promotor eine Basensequenz hat, die die Sequenz TCAATT am 5'-Ende und die Sequenz ACAGTT am 3'-Ende aufweist.

19. Verfahren nach einem der Ansprüche 17 bis 18, bei dem der Promotor die folgende intervenierende, zwischen der 3'- und 5'-Sequenzen befindliche Sequenz aufweist:

ATTAATCATCGAACTAG

20. Verfahren nach Anspruch 16, bei dem der Promotor die folgende Basensequenz aufweist:

TTGACAATTAATCATCGAACTGTTAACT

## Revendications

1. Composition pharmaceutique utile dans la prophylaxie et/ou le traitement d'états pathologiques résultant de la présence de radicaux superoxyde, comprenant une enzyme MnSOD et un excipient acceptable du point de vue pharmaceutique, caractérisée en ce que l'enzyme MnSOD : (i) est sous forme naturelle, (ii) catalyse la dismutation des radicaux superoxyde et (iii) comprend (a) la séquence d'acides aminés de la MnSOD de *Bacillus Stearothermophilus* (BS) ou de *Bacillus Caldotenax* (BC) ou (b) une séquence variante qui diffère des séquences indiquées en (a) par des insertions, délétions et/ou substitutions de 1 à 20 acides aminés.

2. Composition pharmaceutique suivant la revendication 1, dans laquelle l'enzyme MnSOD sous forme naturelle est essentiellement non modifiée chimiquement par rapport à l'enzyme MnSOD présente dans une culture d'un microorganisme BS ou BC produisant une MnSOD.

3. Composition pharmaceutique suivant les revendications 1 ou 2, qui est pratiquement dépourvue de pyrogènes consistant en substances macromoléculaires inhérentes à *B. Stearothermophilus* (BS) ou à *B. Caldotenax.*

4. Composition pharmaceutique suivant l'une quelconque des revendications précédentes, dans laquelle cette enzyme MnSOD est produite par culture d'un microorganisme transformé qui est d'une espèce autre que *B. Stearothermophilus* (BS) ou *B. Caldotenax .*

5. Composition pharmaceutique suivant l'une quelconque des revendications précédentes, dans laquelle cette enzyme MnSOD a une séquence d'acides aminés choisie parmi :
(i) la séquence d'acides aminés représentée dans la figure 12 pour une MnSOD de BC,
(ii) la séquence d'acides aminés représentée dans la figure 3 pour une MnSOD de BS et
(iii) des séquences d'acides aminés qui diffèrent des séquences (i) et (ii) par des insertions, délétions et/ou substitutions de 1 à 10 acides aminés.

6. Composition pharmaceutique suivant la revendication 5, dans laquelle ces séquences d'acides aminés (iii) différent des séquences (i) et (ii) par des insertions, délétions et/ou substitutions de 1 à 5 acides aminés.

7. Composition pharmaceutique suivant l'une quelconque des revendications précédentes sous la forme de (a) une solution injectable, ou (b) une solution convenable pour la perfusion de tissus pendant des procédures chirurgicales ou de transplantation.

8. Composition pharmaceutique suivant la revendication 7, contenant de 0,001 à 1,0 mg/litre de cette enzyme MnSOD.

9. Composition pharmaceutique suivant la revendication 8, contenant de 0,05 à 0,5 mg/litre de cette enzyme MnSOD.

10. Procédé pour la production d'une composition pharmaceutique qui comprend les étapes de : (a) culture d'un microorganisme produisant une enzyme MnSOD de façon à produire une culture contenant de l'enzyme MnSOD, (b) isolement de l'enzyme MnSOD à partir de la culture, (c) purification de l'enzyme MnSOD isolée de façon à produire une enzyme purifiée qui est essentiellement non modifiée chimiquement par rapport à l'enzyme MnSOD présente dans la culture contenant de l'enzyme MnSOD produite dans l'étape (a), et (d) mélange de l'enzyme MnSOD non modifiée chimiquement avec un excipient acceptable du point de vue pharmaceutique, caractérisé en ce que cette enzyme MnSOD : (i) est sous forme naturelle, (ii) catalyse la dismutation des radicaux superoxyde et (iii) comprend (a) la séquence d'acides aminés de la MnSOD de *Bacillus Stearothermophilus* (BS) ou de *Bacillus Caldotenax* (BC) ou (b) une séquence variante qui diffère des séquences indiquées en (a) par des insertions, délétions et/ou substitutions de 1 à 20 acides aminés.

11. Procédé suivant la revendication 10, dans lequel le microorganisme est un microorganisme transformé d'une espèce autre que *B. Stearothermophilus* (BS) ou *B. Caldotenax.*

12. Utilisation d'une enzyme MnSOD dans la fabrication d'une composition pharmaceutique pour la prophylaxie et/ou le traitement d'états pathologiques résultant de la présence de radicaux superoxyde, caractérisée en ce que l'enzyme MnSOD : (i) est sous forme naturelle, (ii) catalyse la dismutation des radicaux superoxyde et (iii) comprend (a) la séquence d'acides aminés de la MnSOD de *Bacillus Stearothermophilus* (BS) ou de *Bacillus Caldotenax* (BC) ou (b) une séquence variante qui diffère des séquences indiquées en (a) par des insertions, délétions et/ou substitutions de 1 à 20 acides aminés.

13. Utilisation d'une enzyme MnSOD dans la fabrication d'une solution de perfusion pour des organes subissant une chirurgie ou une transplantation, en particulier lorsque ces organes sont isolés de la circulation sanguine normale, caractérisée en ce que l'enzyme MnSOD : (i) est sous forme naturelle, (ii) catalyse la dismutation des radicaux superoxyde et (iii) comprend (a) ia séquence d'acides aminés de la MnSOD de *Bacillus Stearothermophilus* (BS) ou de *Bacillus Caldotenax* (BC) ou (b) une séquence variante qui diffère des séquences indiquées en (a) par des insertions, délétions et/ou substitutions de 1 à 20 acides aminés.

14. Enzyme MnSOD étant sous une forme pratiquement pure et ayant la séquence d'acides aminés, ou essentiellement la séquence d'acides aminés de B. *caldotenax,* cette séquence d'acides aminés étant choisie parmi :
(i) la séquence d'acides aminés illustrée dans la figure 12,
(ii) des séquences d'acides aminés qui diffèrent de la séquence (i) par des insertions, délétions et/ou substitutions de 1 à 30 acides aminés, à condition que ces séquences d'acides aminés (ii) aient Glu à la position marquée 103 et/ou aient Ile à la position marquée 188.

15. Molécule d'ADN recombinant comprenant une séquence d'ADN codant pour une enzyme MnSOD telle que définie dans la revendication 14, cette molécule d'ADN recombinant étant choisie de préférence parmi (a) l'ADN codant une séquence illustrée dans la figure 12 et (b) des séquences d'ADN qui sont dégénérées suivant le code génétique pour cette séquence.

16. Procédé pour la production d'une enzyme MnSOD qui comprend la culture d'une souche transformée de E. *coli* contenant un plasmide recombinant comprenant au moins un gène structural codant pour une enzyme MnSOD lié de façon opérative à un promoteur, caractérisé en ce que ce promoteur est le promoteur *trp* naturel de E. *coli.*

17. Procédé suivant la revendication 16, dans lequel ce promoteur a une séquence de bases comprenant la séquence TTGACA à l'extrémité 5' et la séquence TTAACT à l'extrémité 3'.

18. Procédé suivant la revendication 16, dans lequel ce promoteur a une séquence de bases comprenant la séquence TCAATT à l'extrémité 5' et la séquence ACAGTT à l'extrémité 3'.

19. Procédé suivant l'une quelconque des revendications 17 à 18, dans lequel ce promoteur a la séquence intermédiaire suivante située entre ces séquences en 3' et 5':

ATTAATCATCGAACTAG

20. Procédé suivant la revendication 16, dans lequel le promoteur a la séquence de basés suivante :
